# EUROPEAN PATENT APPLICATION

(11) **EP 4 603 588 A2**
(43) Date of publication of application: **20.08.2025**
(21) Application number: 25183717.5
(22) Date of filing: 19.06.2018
(51) Int. Cl.: C12N 15/63

(54) **METHODS AND COMPOSITIONS FOR CONTROLLING CARDIAC FIBROSIS AND REMODELING**

(30) Priority: 19.06.2017 EP 17176725
(62) Divisional of application: 18730384.7
(71) Applicant: Université de Lausanne, 1011 Lausanne (CH)
(72) Inventor: OUZAIN, Samir, 1010 Lausanne (CH); PEDRAZZINI, Thierry, 1052 Le Mont-sur-Lausanne (CH)
(74) Representative: KATZAROV S.A.

(57) **Abstract**

The present invention provides methods and compositions for controlling cardiac fibrosis and heart remodeling in a subject via modulating the expression of one or more lncRNA associated with cardiac-specific super-enhancers (SEs).

## Description

### FIELD OF THE INVENTION

The present invention provides methods and compositions for controlling cardiac fibrosis and heart remodeling in a subject via modulating the expression of one or more lncRNA associated with cardiac-specific super-enhancers (SEs).

### BACKGROUND OF THE INVENTION

Acute myocardial infarction (MI) due to coronary artery disease typically leads to maladaptive myocardial remodeling and heart failure (HF) (*1, 2*). HF places a major economic and clinical burden on the industrialized world, accounting for more than 400,000 deaths and more than 20 billion dollars in annual healthcare costs in the US alone (*3*). Initial translational research has focused on the contracting cells of the heart, the cardiomyocytes (CMs), as a target in therapies aimed at restoring cardiac function. This was despite a wide appreciation that acute and chronic injuries trigger tissue remodeling, which invariably results in and is as a consequence of the development of cardiac fibrosis (*1*). The destruction of the myocardium after infarction is compensated for by the excessive production of extracellular matrix and the formation of a collagen-rich fibrotic scar. Scar formation, tissue remodeling and progressive interstitial fibrosis lead to a severe loss of function and ultimately HF (*1, 2*). Moreover, cross-linking enzymes and posttranslational modifications can alter collagen fibrils. This has important implications for matrix synthesis and degradation, which ultimately determine the onset of diastolic dysfunction (*4*). Despite this clinical importance, very few therapeutic modalities are available to prevent the development of HF. Anti-fibrotic drugs include blockers of the renin-angiotensin-aldosterone system (RAAS) and mineralocorticoid receptor antagonists but are inefficient in the vast majority of fibrotic diseases (*5*). Current medications typically slow the progression of the disease rather than prevent or reverse it, which could be achieved if cardiac fibroblasts (CFs) were the primary cell target (*6*). There is therefore an urgent need to develop alternative therapeutic strategies - for instance, targeting fibroblast differentiation into myofibroblasts or alteration of collagen cross-linking. To achieve this, a deeper characterization of the CF gene program and its associated cellular processes is required to identify specific regulatory molecules and targets (*7, 8*).

Activation and differentiation of CFs into myofibroblasts initiates the pathological process in the diseased heart. Myofibroblasts synthesize and secrete soluble pro-collagen I and III, which are processed by metalloproteinases, cross-linked by lysyl oxidases and hydroxylases, and assembled into dense fibers. The ability of myofibroblasts to resist apoptosis and secrete large quantities of pro-fibrotic signaling molecules contributes to the overall pathogenesis of HF (*1, 6*). Like all differentiated cells, CF identity is hardwired by specific gene regulatory networks (GRNs) (*7*). These GRNs are controlled by core transcription factors (TFs), proteins that interact in a combinatorial manner at cis-regulatory sequences on DNA to regulate downstream programs dictating cell identity and behavior (*9*, *10*)*.* Enhancers, regions of DNA which can be bound by TFs, represent the key information processing units within the genome, and integrate developmental, temporal, spatial and environmental cues (*11*). In addition, enhancers may assemble together, generating large enhancer clusters named super-enhancers (SEs) (*10*, *12*, *13*)*.* These SEs possess important regulatory characteristics, including exquisite cell/tissue-specificity, and appear to be crucial for the maintenance of cell identity. Interestingly, these elements are enriched in single nucleotide polymorphisms (SNPs) linked to common traits and diseases specific to the tissues that harbor them (*12*).

With the recognition that the mammalian genome is predominantly non protein-coding (*15*), the classical protein-centric view of GRN regulation appears to have been premature. RNA-sequencing approaches have revealed that the majority of the noncoding genome is actively transcribed, generating thousands of small and long regulatory noncoding RNAs (ncRNAs) (*15*). Although the implication of microRNAs in the development of stress- and age-induced cardiac fibrosis is well-documented (*16-18*), the more abundant and more diverse long ncRNA (lncRNA) group remains to be comprehensively characterized during heart remodeling. Despite increasing implications in CM hypertrophy and function (*19-22*), the involvement of lncRNAs in regulating cardiac fibrosis needs to be demonstrated (*23*). LncRNAs are able to regulate GRN activity via a disparate array of transcriptional and post-transcriptional mechanisms (*24*). Active enhancers are transcribed into non-coding RNAs, and SEs tend to produce more RNAs than typical enhancers (*25-27*). Enhancer-associated lncRNAs are important for trapping transcription factor proteins on DNA, modifying the local chromatin environment, and organizing nuclear three-dimensional topologies to ensure the correct activation of target gene programs (*27, 28*).

The Inventors recently characterized the long noncoding transcriptome in a murine model of MI and identified hundreds of novel heart-enriched lncRNAs (*29*). Interestingly, the vast majority of these transcripts were associated with active heart-specific enhancers (*29*, *30*), especially those dynamically modulated post-MI. Some of these transcripts were conserved in human and shown to be differentially expressed in cardiac disease including aortic stenosis (AOS) and dilated cardiomyopathy (DCM) (*29*). The Inventors identified *Wisper* (WIsp2 SuPer-Enhancer associated RNA) as a CF-enriched lncRNA that regulates cardiac fibrosis. Of crucial importance, *WISPER* is conserved in human, and its expression in the human heart correlates with collagen content and the severity of cardiac fibrosis. Modulating the expression of this lncRNA highlights the potential for CF-specific SE-associated lncRNAs as therapeutic targets for the amelioration of cardiac fibrosis and ultimately HF.

### SUMMARY OF THE INVENTION

The present invention provides a method for treating and/or preventing a cardiac disease in a subject in need thereof comprising modulating the expression of one or more lncRNA associated with cardiac-specific super-enhancers (SEs).

Also provided is a gene delivery vector comprising i) an endonuclease, and ii) at least one single guide RNA (sgRNA), or crRNA and tracrRNA, targeting a regulatory sequence of an lncRNA associated with cardiac-specific super-enhancers (SEs) or a genomic DNA sequence encoding an lncRNA associated with cardiac-specific super-enhancers (SEs).

Further provided is a method of treating and/or preventing a cardiac disease in a subject in need thereof, said method comprising i) targeting a regulatory sequence of an lncRNA associated with cardiac-specific super-enhancers (SEs) in a cell *ex vivo* by contacting said cell with the gene delivery vector of the invention, wherein the at least one single guide RNA (sgRNA), or crRNA and tracrRNA, directs the endonuclease to and hybridizes to said regulatory sequence, thereby modulating the expression of one or more lncRNA associated with cardiac-specific super-enhancers (SEs), and ii) introducing said cell into the subject, thereby treating and/or preventing said cardiac disease.

Further provided is a method of treating and/or preventing a cardiac disease in a subject in need thereof, said method comprising i) targeting a genomic DNA sequence encoding an lncRNA associated with cardiac-specific super-enhancers (SEs) in a cell *ex vivo* by contacting said cell with the gene delivery vector of the invention, wherein the at least one single guide RNA (sgRNA), or crRNA and tracrRNA, directs the endonuclease to and hybridizes to said genomic DNA sequence, thereby modulating the expression of one or more lncRNA associated with cardiac-specific super-enhancers (SEs), and ii) introducing said cell into the subject, thereby treating and/or preventing said cardiac disease.

Further provided is a composition comprising a gene delivery vector of the invention.

Further provided are pharmaceutical compositions comprising i) a gene delivery vector of the invention, or ii) a cell or population of cells as described herein or iii) a nucleic acid encoding an miRNA, siRNA, piRNA, hnRNA, snRNA, esiRNA, shRNA, or antisense oligonucleotide of the invention,
optionally with one or more pharmaceutically acceptable excipient or carrier.

Also provided is a method of treating and/or preventing a cardiac disease comprising administering a pharmaceutical composition of the invention to a subject in need thereof.

Further provided is a single guide RNA (sgRNA), or crRNA and tracrRNA, targeting a regulatory sequence of one or more lncRNA associated with cardiac-specific super-enhancers (SEs) or a genomic DNA sequence encoding an lncRNA associated with cardiac-specific super-enhancers (SEs).

Further provided is a cell or a population of cells comprising one or more nucleic acid(s) encoding i) an sgRNA, or crRNA and tracrRNA, of the invention1, or ii) an nucleic acid encoding an miRNA, siRNA, piRNA, hnRNA, snRNA, esiRNA, shRNA, or antisense oligonucleotide of the invention, or iii) a gene delivery vector of the invention.

Further contemplated in the present invention are nucleic acids encoding
i) a single guide RNA (sgRNA), or crRNA and tracrRNA, targeting a regulatory sequence of one or more lncRNA associated with cardiac-specific super-enhancers (SEs), or
ii) a single guide RNA (sgRNA), or crRNA and tracrRNA, targeting a genomic DNA sequence encoding an lncRNA associated with cardiac-specific super-enhancers (SEs), or
iii) an miRNA, siRNA, piRNA, hnRNA, snRNA, esiRNA, shRNA, or antisense oligonucleotide targeting a regulatory sequence of one or more lncRNA associated with cardiac-specific super-enhancers (SEs), or
iv) an miRNA, siRNA, piRNA, hnRNA, snRNA, esiRNA, shRNA, or antisense oligonucleotide targeting a genomic DNA sequence encoding an lncRNA associated with cardiac-specific super-enhancers (SEs).

Also provided is a method for controlling cardiac fibrosis and/or heart remodeling in a subject comprising modulating the expression of one or more lncRNA associated with cardiac-specific super-enhancers (SEs).

The invention further contemplates kits for the treatment and/or prevention of a cardiac disease.

### DESCRIPTION OF THE FIGURES

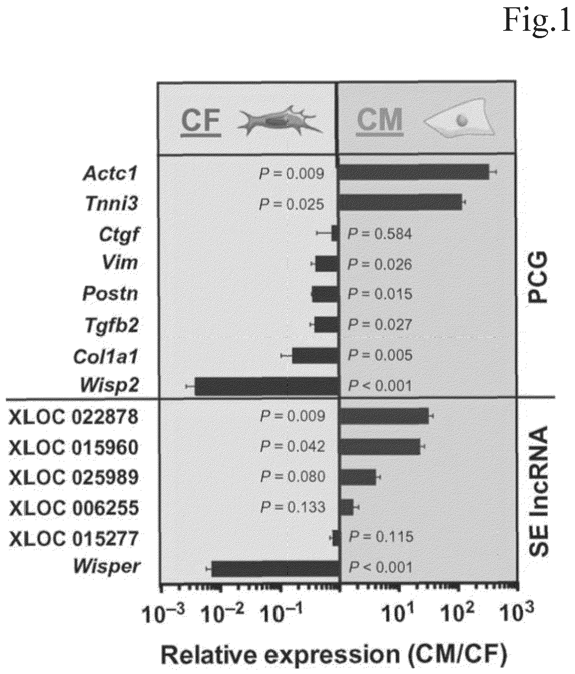

**Figure 1****. Identification of super-enhancer-associated IncRNAs.** qRT-PCR analysis of super-enhancer-associated lncRNAs (SE-lncRNA) and protein coding gene (PCG) expression in cardiomyocytes and fibroblasts isolated from neonatal mouse hearts. Data represent fold change ratio (CM/CF) mean ± SEM (n = 3). *P value* determined by Student's *t* test.

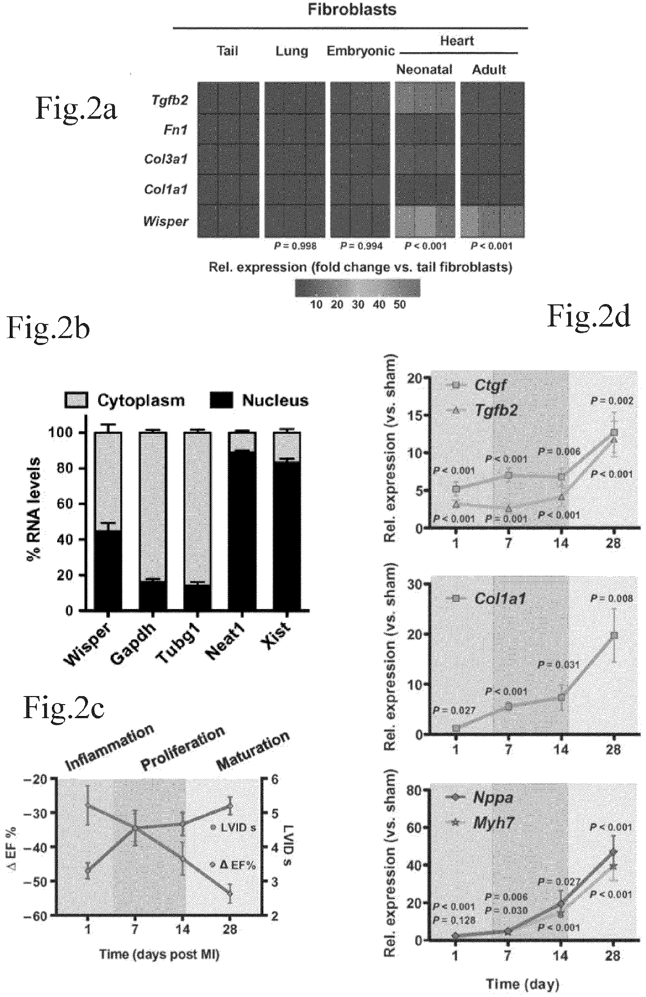

**Figure 2****. *Wisper* is enriched in cardiac fibroblasts and upregulated in fibrotic myocardial tissue.** (A) Heatmap representation of *Wisper* and fibroblast gene expression (relative to tail fibroblasts) in fibroblasts isolated from different tissues. Three independent experiments are shown. *P value* determined by one-way ANOVA (Fisher's test). (B) Percentage of nuclear (black bar) and cytoplasmic (grey bar) RNA concentrations of *Wisper*, *Gapdh* and *Tubg1* (cytoplasmic markers), and *Neat1* and *Xist* (nuclear markers) measured by qRT-PCR after subcellular fractionation in cardiac fibroblasts. Data represent mean ± SEM (n = 4). (C) Ejection fraction (ΔEF; green line) and systolic left ventricular internal dimension (LVID s; blue line) after myocardial infarction by echocardiography as compared to sham. Three different phases of remodeling are highlighted. (D) Expression kinetics of *Wisper* and canonical markers of maladaptive cardiac remodeling measured by qRT-PCR. Graphs show means normalized to sham ± SEM (n = 6 to 10 animals). *P values* determined by two-way ANOVA (Fisher's test).

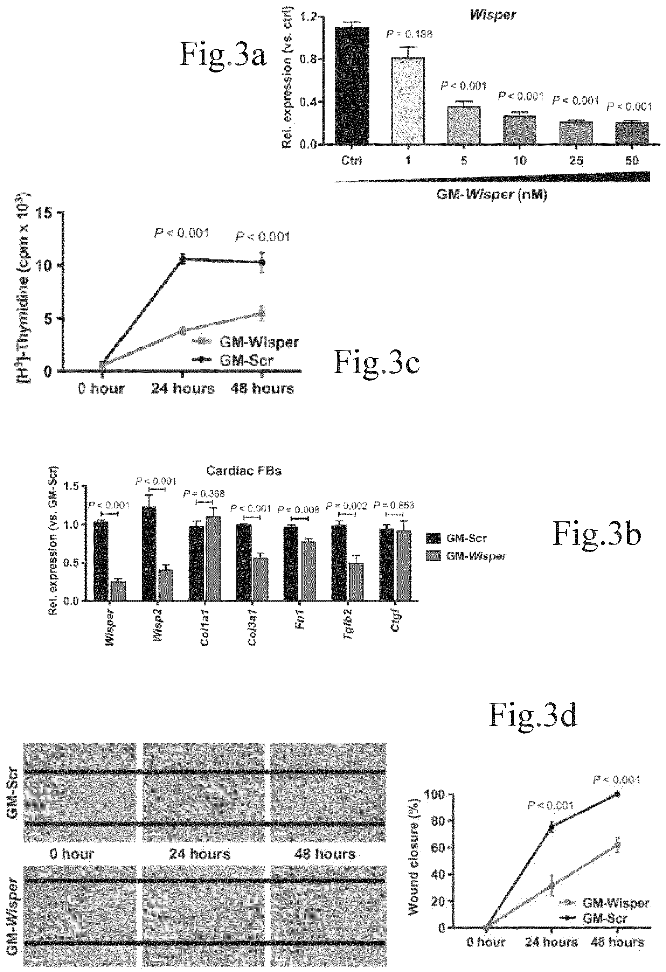

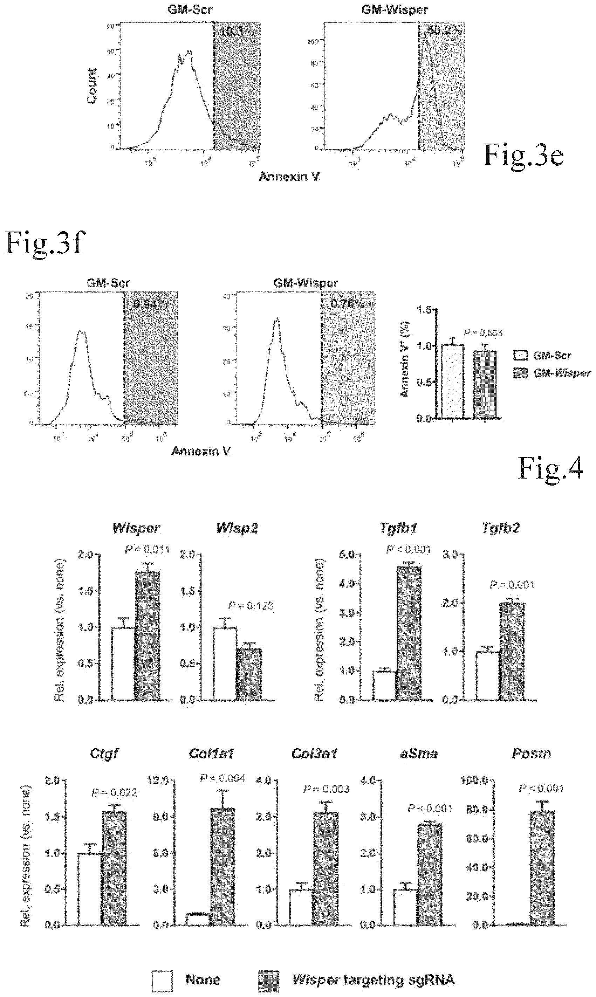

**Figure 3****. *Wisper* controls cardiac fibroblast behavior and survival.** (A) *Wisper* expression in adult cardiac fibroblast (CFs) following GapmeR transfection at increasing concentrations. Bars represent means normalized to control ± SEM (n ≥ 6). *P values* determined by Student's t test. (B) Gene expression measured by qRT-PCR in adult CFs (B) following transfection with GapmeRs (10nM; 48 h; n ≥ 5) targeting *Wisper* (*GM-Wisper*) or scrambled GapmeRs (GM-Scr). *P values* determined by two-way ANOVA (Fisher's test). (C) Proliferation of adult CFs (C) following GapmeR transfection (10 nM) quantified by [H³]-Thymidine incorporation. Data are expressed as mean cpm ± SEM (n ≥ 3). *P values* determined by two-way ANOVA (Bonferroni's test). (D) Migration of adult CFs (D) following GapmeR transfection (10 nM) measured using a wound closure assay. Representative pictures are shown. Scale bar: 100 µM. Graphs show the percentage of wound closure at each time point ± SEM (n ≥ 3). *P values* determined by two-way ANOVA (Bonferroni's test). (E) Representative FACS analysis of annexin V-positive adult lung fibroblasts (E) following GapmeR transfection (10 nM). Graphs show the percentage of apoptotic cells ± SEM (n = 3). *P values* determined by Student's *t* test.
**Figure 4****. Regulation of cardiac fibroblast gene programs by *Wisper.*** Expression of relevant fibrosis-related genes measured by qRT-PCR in P19CL6 cells following CRISPR-on-mediated *Wisper* induction (Wisper targeting sgRNA; grey bar) as compared to control (None; white bar). Mean ± SEM (n=3). *P values* determined by Student's *t* test.

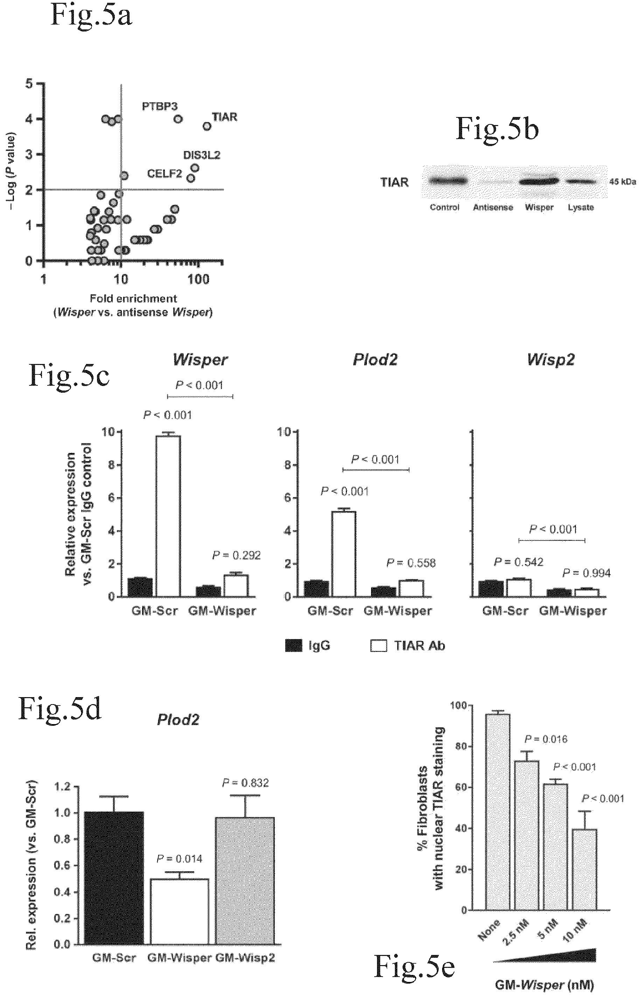

**Figure 5****. Wisper is associated with TIA1-related protein and regulates Lysyl hydroxylase 2 expression.** (A) Proteins enriched following pulldown using biotinylated *Wisper,* and identified by mass spectrometry. The x-axis shows the protein enrichment in the Wisper group as compared to the control antisense *Wisper* group; the y-axis shows the -log P value determined by Fisher's test.) (B) Protein quantification of TIAR by western blotting in the pulled-down protein fraction. Purified TIAR is used as positive control. Graph shows means ± SEM (n = 3). *P values* determined by Student's *t* test. (C) Quantification of RNA following immunoprecipitation using a IgG directed against TIAR or a control IgG. Protein lysate was produced from CFs following transfection with GapmeRs targeting *Wisper* (*GM-Wisper*) or scrambled GapmeRs (GM-Scr), (10 nM, 48 h). Graph shows means ± SEM (n = 6). *P values* determined by two-way ANOVA (Fisher's test). (D) *Plod2* expression in adult CFs following *GM-Wisper*, *GM-Wisp2* or GM-Scr transfection (10 nM, 48 h). Bars represent means ± SEM (n = 3). *P values* determined by one-way ANOVA. (E) Percentage of cardiac fibroblasts with nuclear TIAR staining following *GM-Wisper* treatment at various doses. Bars represent means ± SEM (n > 6). *P values* calculated by two-way ANOVA (Fisher's test).

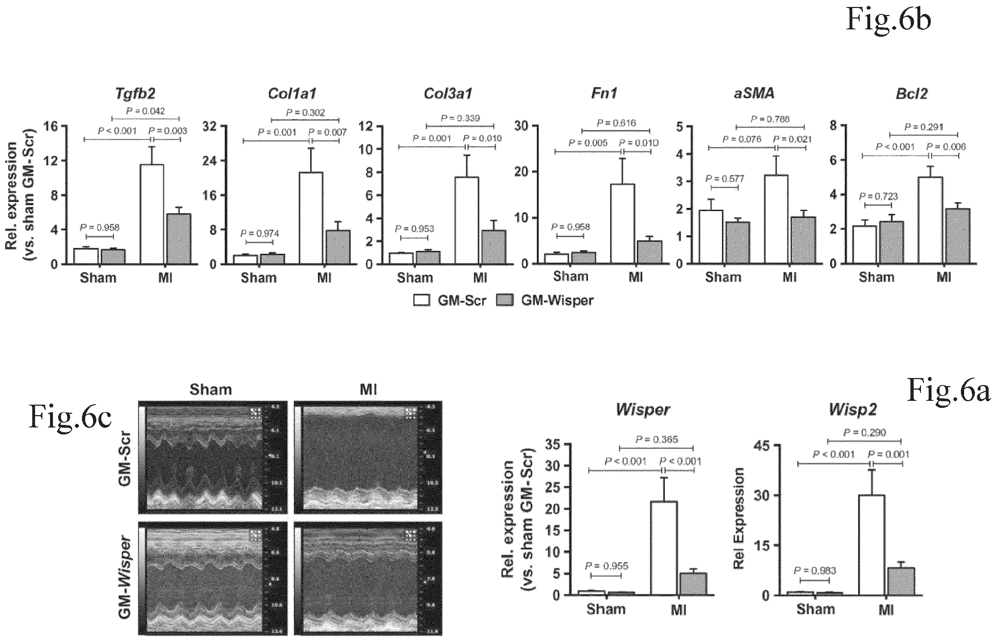

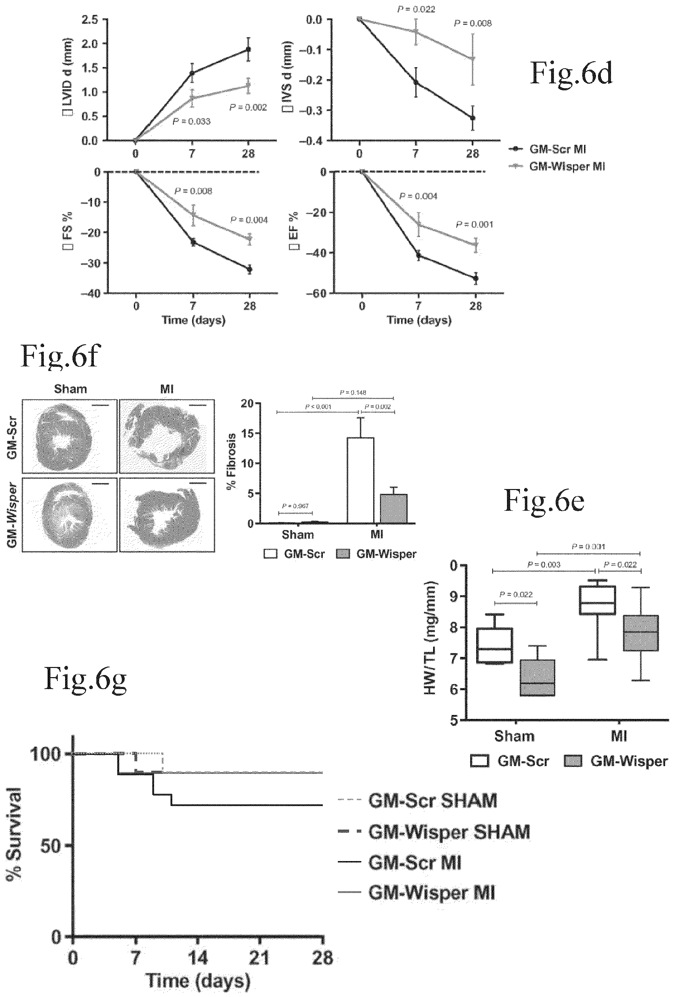

**Figure 6****. Therapeutic depletion of *Wisper* inhibits cardiac fibrosis and improves function.** (A and ) Expression of *Wisper* (A) and fibrotic/stress genes (B) following GapmeR injection (GM-Scr: white; GM-*Wisper*: grey) in sham-operated and MI mice 28 days after surgery. Bars represent means normalized to sham GM-Scr ± SEM. *P values* determined by two-way ANOVA (Fisher's test) (C) M mode images of the left ventricle of GapmeR-injected mice 28 days post-MI. (D) Echocardiographic assessment of cardiac dimension (diastolic left ventricular internal dimension, LVID d; diastolic intra ventricular septum, IVS d) and function (fractional shortening, FS%; ejection fraction, EF%). Graphs show means normalized to the average values of sham ± SEM. *P values* determined by two-way ANOVA (Fisher's test). (E) Heart weight (HW) to tibial length (TL) ratio in GapmeR-injected sham and MI mice. *P values* determined by two-way ANOVA (Fisher's test). (F) Fibrotic tissue quantification by Masson's trichrome staining on heart sections. Graph shows the percentage of cardiac fibrosis as measured by ImageJ (n ≥ 6). Bars represent means normalized to sham GM-Scr ± SEM. *P values* determined by two-way ANOVA (Fisher's test). (I) Effect of GapmeR injection on survival following myocardial infarction.

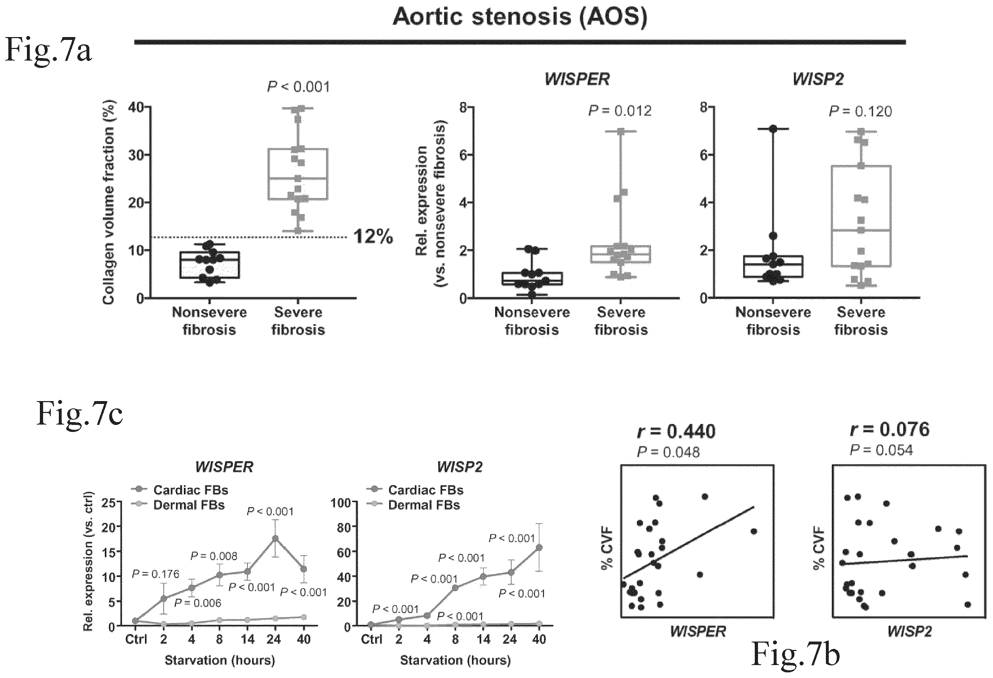

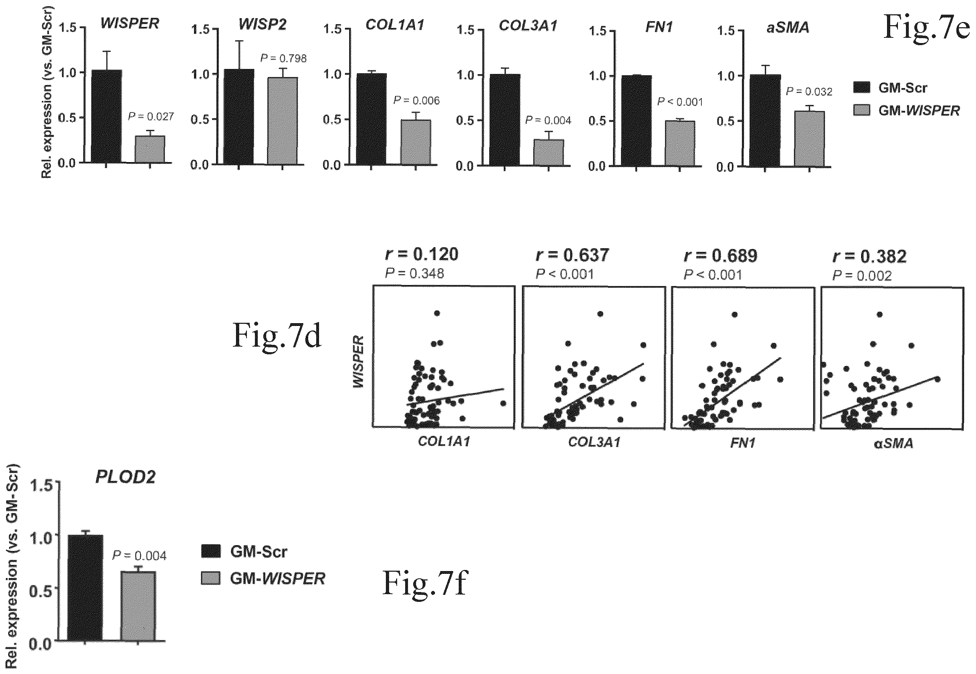

**Figure 7****. *WISPER,* a functionally conserved human ortholog of mouse *Wisper.*** (A) Box plots representation of the collagen volume fraction (CVF) in the heart of the non-severe (n = 11) and severe fibrosis (n = 15) groups of patients affected by aortic stenosis (AOS). The line indicates the CVF cut-off value used differentiate the two groups (12 %). Box plots showing *WISPER* and *WISP2* relative expression analyzed by qRT-PCR in cardiac biopsies from the two different fibrotic groups. Data show the mean, all the individual values and the minimal to maximal variation. *P values* determined by Student's t test (compared to non-severe fibrosis group). (B) Correlation analysis between the CVF and *WISPER* and *WISP2* expression quantified by qRT-PCR. Pearson's correlation test (r; 95% CI). (C) Time course of *WISPER* and *WISP2* expression in differentiating human cardiac fibroblasts (cardiac FBs; red) and dermal FBs (green). Graphs show means normalized to control ± SEM (n = 6 to 14). *P values* vs. control determined by one-way ANOVA. (D) Correlation analysis between *WISPER* expression and *COL1A1, COL3A1, FN1* and *aSMA* expression in differentiating human cardiac fibroblasts. Spearman's correlation test (r; 95% CI). (E) Effect of GapmeR-induced *WISPER* depletion (25 nM, 48 h) on *WISP2* and fibroblast gene expression in human cardiac fibroblasts. Bars show mean ± SEM (n = 3). *P values* determined by Student's *t* test. (F) Effect of GapmeR-induced *WISPER* depletion (25 nM, 48 h) on *PLOD2* expression in human cardiac fibroblasts. Bars show mean ± SEM (n = 3). *P values* determined by Student's *t* test.

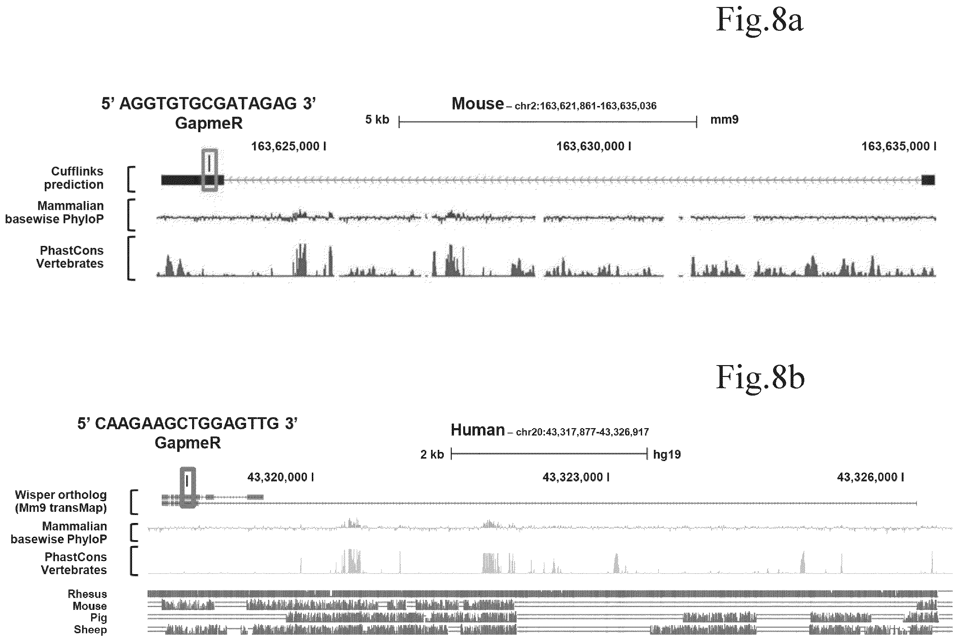

**Figure 8****. Conservation between the mouse and human *Wisper* transcripts.** (A and B) UCSC genome browser views of *Wisper* showing the region targeted by GapmeRs and its sequence in the mouse (A) and human genome (B).

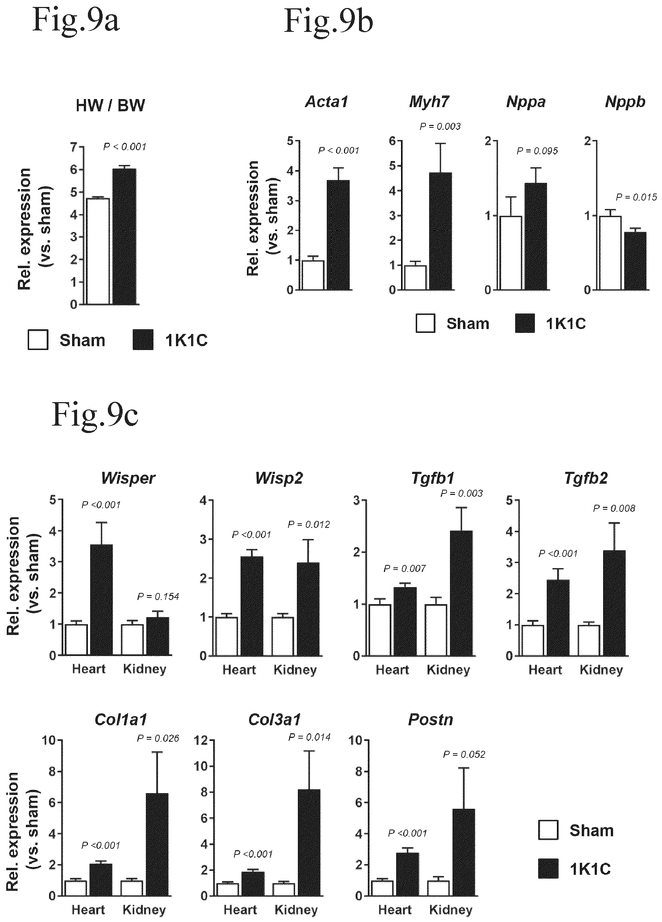

**Figure 9****. *Wisper* is expressed in the stressed fibrotic heart but not in the stressed fibrotic kidney.** (A) Cardiac hypertrophy in mice subjected to the one-kidney one-clip (1K1C) model of renovascular hypertension. 1K1C mice: n=8; Sham-operated mice: n = 7. Heart weight (HW) to tibial length (TL) ratio. Bars represent mean ± SEM. *P values* determined by Student's t test. (B) Cardiac stress marker expression measured by qRT-PCR in sham and 1K1C mice 14 days after surgery. Bars represent means normalized to sham ± SEM. *P values* determined by Student's t test. (C) *Wisper* and fibrosis-associated gene expression measured by qRT-PCR in sham and 1K1C mice. Bars represent means normalized to sham ± SEM. *P values* determined by two-way ANOVA (Fisher's test).

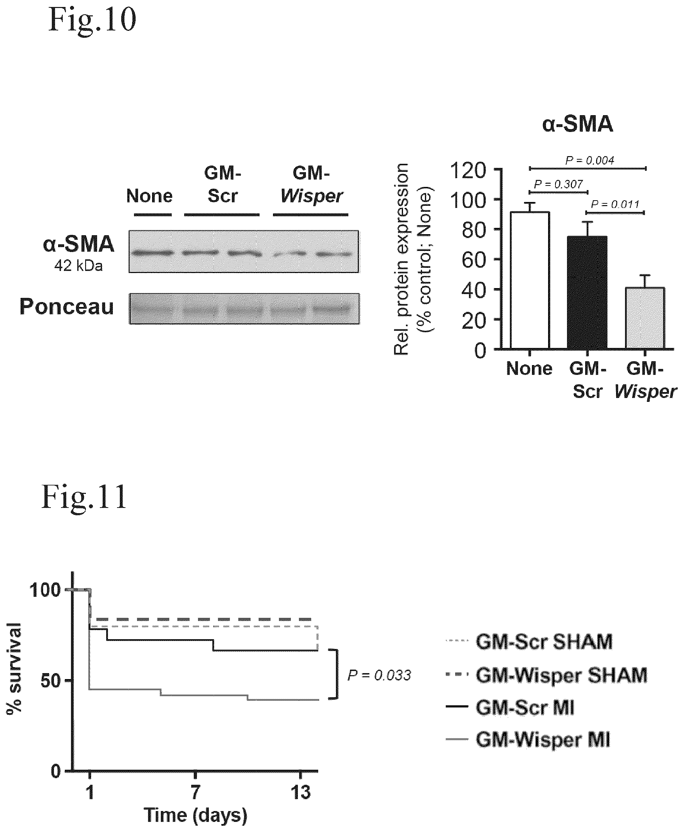

**Figure 10****. Effects of Wisper knockdown in neonatal CFs and CMs.** α-SMA protein quantification by Western blotting in neonatal cardiac fibroblasts. Cells were kept untreated (None) or transfected with scrambled GapmeRs (GM-Scr) or GapmeRs targeting *Wisper* (GM-*Wisper*)*.* Bars represent mean normalized to protein detected by Ponceau staining ± SEM (n ≥ 5). Graph shows mean normalized to untreated cells. *P values* determined by one-way ANOVA.
**Figure 11****. Preventative *Wisper* depletion inhibits cardiac fibrosis and improves function.** Survival after preventative GapmeR injection. *P values* calculated by Log-rank (Mantel-Cox) test vs. the GM-Scr MI group.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components.

As used in the specification and claims, the singular form "a", "an" and "the" include plural references unless the context clearly dictates otherwise.

As used herein, "at least one" means "one or more", "two or more", "three or more", etc.

As used herein the terms "subject"/"subject in need thereof", or "patient"/"patient in need thereof " are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human suffering from a cardiac disease (e.g. cardiac fibrosis after MI) or a human that might be at risk of suffering from a cardiac disease (e.g. MI).

The terms "nucleic acid", "polynucleotide," and "oligonucleotide" are used interchangeably and refer to any kind of deoxyribonucleotide (e.g. DNA, cDNA, ...) or ribonucleotide (e.g. RNA, mRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

The term "vector", as used herein, refers to a viral vector or to a nucleic acid (DNA or RNA) molecule such as a plasmid or other vehicle, which contains one or more heterologous nucleic acid sequence(s) (such as nucleic acid sequence(s) encoding the sgRNA, TRACR and CrRNA, CAS9 nickase, and is designed for transfer between different host cells. The terms "expression vector", "gene delivery vector" and "gene therapy vector" refer to any vector that is effective to incorporate and express one or more nucleic acid(s), in a cell, preferably under the regulation of a promoter. A cloning or expression vector may comprise additional elements, for example, regulatory and/or post-transcriptional regulatory elements in addition to a promoter.

The term "about," particularly in reference to a given quantity, is meant to encompass deviations of plus or minus ten (10) percent.

After MI, activated CFs and associated ECM production assume crucial roles during the acute and chronic phases of the adaptive response of the heart to new hemodynamic conditions (1). However, maladaptive changes in ECM dynamics lead to the long term disruption of myocardial architecture and function, eventually leading to heart failure. Excessive ECM deposition and its adverse effects are potentially modifiable (*5, 34*); indeed, a reduction in the development of fibrosis can be observed in humans treated with therapeutics aimed at limiting pathological remodeling of the diseased heart (*5*). Due to the non-targeted nature of these approaches, beneficial effects on fibrosis are relatively modest. Nonetheless, these agents improved clinical outcomes providing a strong rationale for the development of highly specific therapeutics directly targeting the CF population.

While focusing on the enhancer landscape and their associated transcripts, the Inventors surprisingly discovered the evolutionary conserved super-enhancer (SE)-associated lncRNAs. One of them, which was named *Wisper,* is a polyadenylated and multiexonic, CF-enriched transcript. Although the human genome encodes thousands of polyadenylated multiexonic lncRNAs, their functional and translational importance in controlling pathological remodeling and fibrosis remain largely unexplored.

Preferably, the one or more lncRNA associated with cardiac-specific super-enhancers (SEs) of the invention are human CF-enriched transcripts. Most preferably, the one or more lncRNA associated with cardiac-specific super-enhancers (SEs) of the invention are selected from the group comprising *Wisper*, *Sparc*, *Col15A1*, *Cyr61*, *Smad7* and a combination of one or more thereof. More preferably, the lncRNA associated with cardiac-specific super-enhancers (SEs) is *Wisper.*

The inventors have shown that *Wisper* depletion leads to a global transcriptional reprogramming of the networks controlling proliferation, migration, apoptosis and differentiation solely in CFs. Considering its distribution within both the cytoplasm and nucleus, *Wisper* could exert its action via both *cis-* and *trans*-regulatory functions, presumably-*but without wishing to be bound by the theory-*via interaction with nuclear and cytoplasmic RNA binding proteins. In this context, the Inventors identified TIAR as a *Wisper*-associated protein. TIAR plays important role in strengthening alternative 5' splice sites (*49*). PLOD2 has two splice variants with the long form containing an additional exon, whose inclusion is controlled by TIAR. TIAR has been therefore implicated in tissue fibrosis via its capacity to promote production of the long pro-fibrotic PLOD2 form (*44*). The short form is not expressed in the heart. Moreover, both *Wisper* and *Plod2* bind TIAR, and downregulation of *Wisper* in CFs decreases *TIAR*/*Plod2* interaction and *Plod2* expression, suggesting that *Wisper* regulates *Plod2* mRNA posttranscriptional processing and its cellular concentrations via controlling *Plod2* association with TIAR. Interestingly, PLOD2 has also been shown to induce collagen synthesis independently of its action on crosslinking and stabilization of the matrix (*50*), further supporting a central role for TIAR and PLOD2 in fibrosis. In addition, TIAR is a multifunctional protein that dictates many aspects of RNA metabolism. TIAR functionally interacts with noncoding RNAs, including lncRNAs (*51*, *52*). LncRNA-mediated regulation of TIAR is therefore emerging as a common mechanism to confer context and cell specificity to an otherwise ubiquitously acting system. In particular, TIAR has been implicated in the control of cell proliferation and apoptosis. Remarkably, the GO terms associated with the transcriptional programs following *Wisper* knockdown are highly reminiscent to the GO terms associated with modulated genes in response to TIAR knockdown (*53*). In this context, *Wisper* functions in part by controlling TIAR shuttling into the nucleus (45). Blocking nuclear translocation of TIAR in *Wisper-depleted* cells is therefore expected to produce global effects on fibroblast gene programs. Three other proteins were identified by mass spectrometry following *Wisper* pulldown. PTBP3, DIS3L2 and CELF2 demonstrate relevant functions as regulators of differentiation, proliferation and apoptosis *(41-43).* Their roles in cardiac fibroblasts, and in particular in the development of fibrosis, have not been investigated. These candidates warrant therefore further characterization. It is important to note, however, that many lncRNAs are associated with RNA processing factors involved in maturation of the primary transcripts via posttranscriptional modification. Association of these proteins with a multiexonic lncRNA such as *Wisper* might also reflect the need for this transcript to be appropriately processed for ensuring its functions. In this regard, TIAR, while being a splicing factor, is the only protein of this small group with a demonstrated link to fibrosis.

The extent of gene reprogramming in fibroblast following *Wisper* knockdown demonstrate that *Wisper* exerts several important functions, some of them being probably independent of TIAR. The Inventors examined therefore expression of genes topologically associated within the *Wisper* locus. Among those, *Wisp2* is co-modulated with *Wisper* during fibroblast differentiation and after *Wisper* knockdown, suggesting that *Wisper* could directly control its proximal coding gene in *cis.* However, several pieces of evidence indicate that concomitant expression of *Wisper* and *Wisp2* does not necessarily reflect *cis-*regulation. Although *Wisper* knockdown in mouse CFs in vitro induces *Wisp2* downregulation, this effect is not observed in human CFs. In addition, the expression signature after *Wisp2* loss of function in CFs is distinct from that observed after *Wisper* knockdown, suggesting that *Wisper* effects are not mediated via *Wisp2.* Moreover, CRISPR-on-mediated induction of *Wisper* expression at its site of transcription does not activate *Wisp2* expression. Finally, *Wisp2* is highly expressed in the lungs where modest *Wisper* expression is observed. The reason for *Wisp2* being highly expressed in this organ is possibly related to the presence in its promoter of binding sites for transcription factors that are known to induce lung-specific gene programs. In the heart, *Wisp2* has recently been implicated in the therapeutic regression of cardiac fibrosis (*31*). *Wisp2* appears to act therefore more as a compensatory molecule that limits the extent of fibrosis rather than a promoter of fibrosis. We believe therefore that *Wisp2* downregulation in the heart occurs secondary to *Wisper* depletion as a direct consequence of the induced beneficial impact on cardiac fibrosis.

Unlike most tissues in which myofibroblasts undergo apoptosis or revert back to a quiescent state in the absence of pathological stress, this does not occur in the heart. However, GapmeR-mediated depletion of *Wisper* prior to MI attenuates pathological fibrosis and remodeling. Nevertheless, preventive *Wisper* depletion also negatively impacts the acute wound healing process, resulting in cardiac rupture and increased mortality. Loss of CFs in the stressed heart affects the matrix that is key for the homeostatic maintenance of myocardial integrity (*6*). CFs produce the collagenous matrix that prevents myofiber slippage and sustains ventricular chamber geometry under normal conditions; interfering with this process results in weakening of the ventricular wall and susceptibility to rupture. Along the same lines, it is likely that massive doses of anti-Wisper GapmeRs destabilize the delicate architecture of the normal heart, leading to cardiac dysfunction.

The detrimental effects of the *Wisper* GapmeR treatment during the acute phase could be overcome when using a therapeutic protocol in which GapmeRs were administered 2 days post-infarction. *Wisper* knockdown during this clinically relevant window of time did not negatively affect acute wound healing while still blunting pathological fibrosis, reducing remodeling and improving cardiac function at 7 and 28 days post-infarction. These data coupled with the observation that human *WISPER* expression is correlated with fibrosis in AOS patients support translation into clinical scenarios. Clearance of activated CFs from the diseased heart appears to be a highly efficient process leading to progressive reduction of pathological fibrosis and diminished incidence of HF (*5*, *6*). One could also envisage Wisper being targeted in the context of unchecked reactive fibrosis in the myocardium independently of the underlying disease etiology. This could be of high clinical relevance as myocardial fibrosis persists in patients with HF even when treated according to current guidelines, and correlates with mortality. An important finding in the present study is the apparent cardiac specificity of *Wisper* expression. Under basal conditions, *Wisper* is clearly a cardiac fibroblast-enriched transcript. Cardiac TF binding sites in the SE element provides an explanation for this interesting feature. Finally, our data also demonstrate that lncRNA associated with cardiac-specific super-enhancers (SEs) like *Wisper* are potentially highly sensitive markers for pathological fibrosis. Considering the ability to detect lncRNAs circulating in human plasma (*55*), measurement of circulating *WISPER* concentrations might provide a noninvasive means to monitor cardiac remodeling. Interestingly, we have previously analyzed a series of novel lncRNAs including *Wisper,* and correlated their expression with echocardiographic traits after infarction, supporting the notion that these transcripts may represent interesting marker candidates (*29, 66*). Altogether, the identification of this CF-specific regulatory molecule represents therefore an important step toward the development of targeted anti-fibrotic therapeutic approaches and diagnostic tools.

The present invention thus concerns a method for treating and/or preventing a cardiac disease in a subject in need thereof, said method comprising modulating the expression of one or more lncRNA associated with cardiac-specific super-enhancers (SEs).

The term "treatment" or "treating" means any administration of a composition, pharmaceutical composition, therapeutic agent, compound, *etc...* of the disclosure to a subject for the purpose of:
(i) inhibiting the disease, that is, arresting the development of clinical symptoms; and/or
(ii) relieving the disease, that is, causing the regression of clinical symptoms.

As used herein, the term "prevention" or "preventing" means any administration of a composition, pharmaceutical composition, therapeutic agent, compound, *etc...* of the disclosure to a subject for the purpose of:
(i) preventing the disease, that is, causing the clinical symptoms of the disease not to develop;

In the context of the present invention, the disease is a cardiac disease, preferably a cardiac fibrosis.

The expression of the one or more lncRNA associated with cardiac-specific super-enhancers (SEs) can be either upregulated or downregulated. Preferably, the expression of the one or more lncRNA associated with cardiac-specific super-enhancers (SEs) is modulated by repressing or activating the transcription of said lncRNAs.

Any suitable method for modulating the expression of one or more lncRNA associated with cardiac-specific super-enhancers (SEs) may be used that results in either upregulation or downregulation of said expression. Examples of methods include RNAi mediated knock-down using antisense oligonucleotides (ASOs) and gene editing systems.

Any nuclease of the gene editing system known in the art, such as a meganuclease, zinc finger nuclease (ZFNs), transcription activator-like effector-based nuclease (TALEN), CPF1 is also intended to be within the scope of the present invention.

ASOs may be directed against any cis-regulatory or trans- regulatory sequences, or fragments thereof, or variants thereof, of the one or more lncRNA of the invention. Alternatively, ASOs may also be directed against a genomic sequence encoding an lncRNA of the invention.

Examples of ASOs include, e.g., miRNA, siRNA, piRNA, snRNA or a modified ASO such as GapmeRs.

The terms "microRNA," "miRNA," and MiR" are interchangeable and refer to endogenous or artificial non-coding RNAs that are capable of regulating gene expression. It is believed that miRNAs function via RNA interference. The terms "siRNA" and "short interfering RNA" are interchangeable and refer to single-stranded or double-stranded RNA molecules that are capable of inducing RNA interference. SiRNA molecules typically have a duplex region that is between 18 and 30 base pairs in length.

The terms "piRNA" and "Piwi-interacting RNA" are interchangeable and refer to a class of small RNAs involved in gene silencing. PiRNA molecules typically are between 26 and 31 nucleotides in length.

The terms "snRNA" and "small nuclear RNA" are interchangeable and refer to a class of small RNAs involved in a variety of processes including RNA splicing and regulation of transcription factors. The subclass of small nucleolar RNAs (snoRNAs) is also included. The term is also intended to include artificial snRNAs, such as antisense derivatives of snRNAs comprising antisense sequences directed against one or more lncRNAs.

Examples of modified ASOs include the GapmeRs. As used herein, a GapmeR is a chimeric antisense oligonucleotide that contains a central block of deoxynucleotide monomers sufficiently long to induce RNase H cleavage. Usually, the GapmeRs of the invention are directed against one or more lncRNA sequences. Preferably, the GapmeR is selected from the group comprising SEQ ID No. 12, SEQ ID No. 14 or a combination thereof.

Preferably, the expression of one or more lncRNA is upregulated or downregulated by using a gene editing system such as, e.g. the CRISPR-based gain/loss-of-function system. Usually, the CRISPR-based gain/loss-of-function system comprises at least one single guide RNA (sgRNA), or crRNA and tracrRNA, and a structure-guided endonuclease such as an RNA-guided endonuclease.

Any suitable naturally occurring, or engineered, RNA-guided endonuclease can be employed as long as it is effective for binding a target DNA and it may be selected from the non-limiting group comprising Cas9, Cpf1, and FEN-1. Preferably, the RNA-guided endonuclease is Cas9.

The CRISPR/Cas9 system has become a remarkably flexible tool for genome manipulation over the years. A unique feature of Cas9 endonuclease is its ability to bind target DNA independently of its ability to cleave target DNA.

Within the context of this disclosure, the Cas9 endonuclease is preferably a modified Cas9 endonuclease such as, e.g. an enzymatically dead Cas9. Specifically, both RuvC- and HNH-nuclease domains can be rendered inactive by point mutations (e.g. D10A and H840A in SpCas9), resulting in a nuclease dead Cas9 molecule that cannot cleave target DNA. However, the dead Cas9 molecule retains the ability to bind to target DNA based on the sgRNA targeting sequence, which sgRNA sequence is comprised in CRISPR-based gain/loss-of-function system.

In one aspect, the enzymatically dead Cas9 is tagged with one or more transcriptional repressor or one or more transcriptional activator (see (*68*) which is incorporated herein by reference).

In another aspect, the enzymatically dead Cas9 is tagged with one or more epitope that is/are recognized by one or more antibody-activator/repressor effector. This enzymatically tagged dead Cas9 can then target the regulatory sequence resulting in robust transcription repression or activation of downstream target gene encoding the lncRNA of the invention.

Usually and within the context of the invention, the regulatory sequence is a promoter or enhancer region which is either a *cis*-or a *trans*-acting regulatory sequence. Preferably, said regulatory sequence is selected from the non-limiting group comprising the proximal Wisper enhancer (SEQ ID No. 1 ), the proximal Wisper promoter (SEQ ID No. 2), the Wisper super-enhancer (SEQ ID No. 3), the Wisper downstream cis sequence (SEQ ID No. 4), the Smad7 cis-regulatory sequence (SEQ ID No. 5), the Cyr61 cis-regulatory sequence (SEQ ID No. 6), the Col15A1 cis-regulatory sequence (SEQ ID No. 7), the Sparc cis-regulatory sequence (SEQ ID No. 8), or fragments thereof, or variants thereof, and a combination of one or more thereof. Most preferably, the regulatory sequence is selected from the non-limiting group comprising the proximal Wisper enhancer (SEQ ID No. 1), the proximal Wisper promoter (SEQ ID No. 2), the Wisper super-enhancer (SEQ ID No. 3), the Wisper downstream cis sequence (SEQ ID No. 4), or fragments thereof, or variants thereof, and a combination of one or more thereof.

The at least one single guide RNA (sgRNA), or crRNA and tracrRNA, usually recognizes a target sequence comprising 16 to 25 nucleotides.

As described below, the target sequence is a DNA regulatory sequence of an lncRNA associated with cardiac-specific super-enhancers (SEs). Alternatively, the target sequence is a DNA, e.g. genomicDNA, encoding for an lncRNA associated with cardiac-specific super-enhancers (SEs). Preferably, the lncRNA associated with cardiac-specific super-enhancers (SEs) is *Wisper.*

Any suitable engineered sgRNA, or crRNA and tracrRNA, can be employed as long as it is effective for recognizing a target DNA of the invention. The design of such sgRNA, or crRNA and tracrRNA is within the skill of ordinary artisans. The sgRNA can, e.g. be the sgRNA MS2 described herein as set forth in SEQ ID No. 13.

Usually, the method for treating and /or preventing a cardiac disease in a subject in need thereof comprises the step of administering a gene delivery vector or an acid nucleic as described herein.

"Administering", as it applies in the present invention, refers to contact of an effective amount of a gene delivery vector or an acid nucleic of the invention, to the subject.

Administering a nucleic acid of the invention, such as ASOs (e.g. miRNA, siRNA, piRNA, snRNA) or modified ASOs (GapmeRs) to a cell comprises transducing, transfecting, electroporating, translocating, fusing, phagocytosing, shooting or ballistic methods, etc., i.e., any means by which a nucleic acid can be transported across a cell membrane.

Another aspect of the invention concerns a gene delivery vector comprising
i) an endonuclease of the invention, and
ii) at least one single guide RNA (sgRNA), or crRNA and tracrRNA, recognizing a target sequence comprising 16 to 25 nucleotides.

Any suitable vector can be employed that is effective for introduction of one or more nucleic acid(s) into cells. Preferably, the gene delivery vector of the invention is a viral vector, such as a lenti- or baculo- or preferably adeno-viral/adeno-associated viral (AAV) vectors, but other means of delivery or vehicles are known (such as yeast systems, microvesicles, gene guns/means of attaching vectors to gold nanoparticles) and are provided, in some aspects, one or more of the viral or plasmid vectors may be delivered via liposomes, nanoparticles, exosomes, microvesicles, or a gene-gun. Most preferably, the gene delivery vector is selected from the group comprising an adeno-associated virus (AAV) and a lentivirus. Lentivirus of 1st, 2nd, and 3rd generation are also envisioned and are known in the art.

The type of AAV surface protein determines the target tissue. Preferably, any adeno -associated virus (AAV) serotype or engineered AAV is envisioned. Most preferably, the AAV will be selected from the group comprising AAV6 and AAV9, due to their broad tissue specificity and expression levels. AAV9 particularly has a minimal inflammatory response, thereby reducing the side effects. The viral particles will usually be administered by injection into the bloodstream. AAV6 can also be used with cultured patient-derived cells. This will be useful, e.g. when using the approach in iPSCs or ES cells as described in the present disclosure.

Preferably, the endonuclease is a Cas9 endonuclease, most preferably a modified Cas9 endonuclease such as, e.g. an enzymatically dead Cas9.

In one aspect, the enzymatically dead Cas9 is optionally tagged with one or more transcriptional repressor or one or more transcriptional activator depending on whether the expression, i.e. the transcription, of the one or more lncRNA of the invention is to be repressed or activated. Transcription repression by dCas9 can be improved by fusing dCas9 with different repressor domains including, e.g. MAX-interacting protein 1 (MXI1), Krüppel-associated box (KRAB) domain or four concatenated mSin3 domains (SID4X), to either amino or carboxyl termini. On the other hand, transcription activation can be improved by fusing dCas9 with different activator domains including, e.g., multiple repeats of the herpes simplex VP16 activation domain (VP64 or VP160) or the nuclear factor-κB (NF-κB) transactivating subunit activation domain (p65AD) (see (*67*) Dominguez et al., 2016 which is incorporated herein by reference).

In another aspect, the enzymatically dead Cas9 is optionally tagged with one or more epitope that is/are recognized by one or more antibody-activator/repressor effector. This enzymatically tagged dead Cas9 can then target the regulatory sequence resulting in robust transcription repression or activation of downstream target gene encoding the lncRNA of the invention.

Usually, said "target sequence" is a regulatory sequence of an lncRNA associated with cardiac-specific super-enhancers (SEs). Typically, this target sequence will be selected from the lncRNA regulatory sequences listed in Table 1.

A further aspect of the invention relates to a method of treating and/or preventing a cardiac disease in a subject in need thereof, said method comprising
(a) targeting a regulatory sequence of an lncRNA associated with cardiac-specific super-enhancers (SEs) in a cell *ex vivo* by contacting said cell with a gene delivery vector of the invention, wherein the at least one single guide RNA (sgRNA), or crRNA and tracrRNA, directs a structure-guided endonuclease of the invention, such as a Cas9 endonuclease, and (b) introducing said cell into the subject, thereby treating and/or preventing said cardiac disease.

Preferably, the Cas9 is a modified Cas9 endonuclease such as, e.g. an enzymatically dead Cas9. Most preferably, said Cas9 (e.g. an enzymatically dead Cas9) is modified for transcriptional activation and/or repression and hybridizes to regulatory sequence, thereby modulating the expression of one or more lncRNA associated with cardiac-specific super-enhancers (SEs),

Preferably, the cardiac disease is cardiac fibrosis.

The invention further relates to pharmaceutical compositions.

In one aspect, the pharmaceutical composition comprises a gene delivery vector of the invention, preferably in an effective amount. The pharmaceutical composition may further comprise one or more pharmaceutically acceptable excipient or carrier.

In another aspect, the pharmaceutical composition comprises a nucleic acid (e.g. an ASO or a modified ASO such as a GapmeR) of the invention, preferably in an effective amount. The pharmaceutical composition may further comprise one or more pharmaceutically acceptable excipient or carrier.

An "effective amount", when it refers to an active principle of a pharmaceutical composition of the invention, is an amount sufficient to effect beneficial or desired results, such as an amount that inhibits the activity of an lncRNA, for example by interfering with transcription. An effective amount can be administered in one or more administrations, applications, or dosages.

In a further aspect, the pharmaceutical composition comprises a nucleic acid (e.g. an ASO or a modified ASO) targeting an lncRNA associated with cardiac-specific super-enhancers (SEs). Most preferably, said one or more antisense oligonucleotide targets *Wisper* and is a modified antisense oligonucleotide (GapmeR) having a sequence as set forth in SEQ ID No 12 or 14 or a combination thereof.

A "Pharmaceutically acceptable excipient or carrier" refers to an excipient that may optionally be included in the compositions of the invention and that causes no significant adverse toxicological effects to the patient. It usually refers to a diluent, adjuvant, or vehicle with which the active principle is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. A thorough discussion of pharmaceutically acceptable excipients is available in REMINGTON'S PHARMACEUTICAL SCIENCES (Mack Pub. Co., N.J. 1991) which is incorporated by reference herein.

"Pharmaceutically acceptable salt", e.g. of a nucleic acid of the invention, includes, but is not limited to, amino acid salts, salts prepared with inorganic acids, such as chloride, sulfate, phosphate, diphosphate, bromide, and nitrate salts, or salts prepared from the corresponding inorganic acid form of any of the preceding, e.g., hydrochloride, etc., or salts prepared with an organic acid, such as malate, maleate, fumarate, tartrate, succinate, ethylsuccinate, citrate, acetate, lactate, methanesulfonate, benzoate, ascorbate, para-toluenesulfonate, palmoate, salicylate and stearate, as well as estolate, gluceptate and lactobionate salts. Similarly salts containing pharmaceutically acceptable cations include, but are not limited to, sodium, potassium, calcium, aluminum, lithium, and ammonium (including substituted ammonium).

Another aspect of the invention concerns a method of treating and/or preventing a cardiac disease comprising administering a pharmaceutical composition of the invention to a subject in need thereof. Preferably, a pharmaceutical composition of the invention is administered in a therapeutically effective dose or amount. A pharmaceutical composition of the invention can be administered alone or in combination with one or more additional therapeutic agents.

By "therapeutically effective dose or amount" is intended an amount of a therapeutic agent that, when administered as described herein, brings about a positive therapeutic response. The exact amount required will vary from subject to subject, depending on the species, age, and general condition of the subject, the severity of the condition being treated, the particular drug or drugs employed, mode of administration, and the like. An appropriate "effective" amount in any individual case may be determined by one of ordinary skill in the art using routine experimentation, based upon the information provided herein. Preferably, the cardiac disease is cardiac fibrosis.

The actual dose to be administered will vary depending upon the therapeutic agent (gene delivery vector or nucleic acid), age, weight, and general condition of the subject as well as the severity of the condition being treated, the judgment of the health care professional, and conjugate being administered. Therapeutically effective amounts can be determined by those skilled in the art, and will be adjusted to the particular requirements of each particular case.

Generally, a therapeutically effective amount of a nucleic acid of the invention, will range from about 0.50 mg to 5 grams daily, more preferably from about 5 mg to 2 grams daily, even more preferably from about 7 mg to 1.5 grams daily.

In certain aspects, multiple therapeutically effective doses of each of at least one nucleic acid and at least one additional therapeutical agent will be administered according to a daily dosing regimen, or intermittently. For example, a therapeutically effective dose can be administered, one day a week, two days a week, three days a week, four days a week, or five days a week, and so forth. By "intermittent" administration is intended the therapeutically effective dose can be administered, for example, every other day, every two days, every three days, and so forth. By "twice-weekly" or "two times per week" is intended that two therapeutically effective doses of the agent in question is administered to the subject within a 7 day period, beginning on day 1 of the first week of administration, with a minimum of 72 hours, between doses and a maximum of 96 hours between doses. By "thrice weekly" or "three times per week" is intended that three therapeutically effective doses are administered to the subject within a 7 day period, allowing for a minimum of 48 hours between doses and a maximum of 72 hours between doses. For purposes of the present invention, this type of dosing is referred to as "intermittent" therapy. In accordance with the methods of the present invention, a subject can receive intermittent therapy (i.e., twice-weekly or thrice-weekly administration of a therapeutically effective dose) for one or more weekly cycles until the desired therapeutic response is achieved. The agents can be administered by any acceptable route of administration as noted herein below.

In case the pharmaceutical composition of the invention comprises a GapmeR targeting an lncRNA associated with cardiac-specific super-enhancers (SEs), then it will preferably be administered 1-5 days post-infarction, preferably 2-4 days post-infarction, most preferably 2 days post-infarction.

When the pharmaceutical composition comprising a GapmeR of the invention is to be administered for preventing a cardiac disease, then said pharmaceutical composition of the invention will preferably be administered before the risk of myocardial infarction.

More preferably, said one or more antisense oligonucleotide targets *Wisper* and is a modified antisense oligonucleotide (GapmeR) having a sequence as set forth in SEQ ID No 12 or 14.

A therapeutic agent of the invention can be administered prior to, concurrent with, or subsequent to at least one additional therapeutic agent. If provided at the same time as the additional therapeutic agent, the active agent can be provided in the same or in a different composition. Thus, the agents can be presented to the individual by way of concurrent therapy. By "concurrent therapy" is intended administration to a human subject such that the therapeutic effect of the combination of the substances is caused in the subject undergoing therapy. For example, concurrent therapy may be achieved by administering at least one therapeutically effective dose of a pharmaceutical composition comprising an active agent and at least one therapeutically effective dose of a pharmaceutical composition comprising at least one additional therapeutic agent according to a particular dosing regimen. Administration of the separate pharmaceutical compositions can be at the same time (i.e., simultaneously) or at different times (i.e., sequentially, in either order, on the same day, or on different days), so long as the therapeutic effect of the combination of these substances is caused in the subject undergoing therapy.

In other aspects of the invention, the pharmaceutical composition of the invention is a sustained-release formulation, or a formulation that is administered using a sustained-release device. Such devices are well known in the art, and include, for example, transdermal patches, and miniature implantable pumps that can provide for drug delivery over time in a continuous, steady-state fashion at a variety of doses to achieve a sustained-release effect with a non-sustained-release pharmaceutical composition. The pharmaceutical compositions of the invention may be administered using the same or different routes of administration in accordance with any medically acceptable method known in the art. Suitable routes of administration include parenteral administration, such as subcutaneous (SC), intraperitoneal (IP), intramuscular (IM), intravenous (IV), or infusion, oral and pulmonary, nasal, topical, transdermal, and suppositories. Where the composition is administered via pulmonary delivery, the therapeutically effective dose is adjusted such that the soluble level of the agent is equivalent to that obtained with a therapeutically effective dose that is administered parenterally, for example SC, IP, IM, or IV. In some embodiments of the invention, the pharmaceutical composition is administered by IM or SC injection, particularly by IM or SC injection locally to the region where the therapeutic agent or agents used in the cardiac therapy protocol are administered.

Factors influencing the respective amount of the various compositions to be administered include, but are not limited to, the mode of administration, the frequency of administration (i.e., daily, or intermittent administration, such as twice- or thrice-weekly), the particular disease undergoing therapy, the severity of the disease, the history of the disease, whether the individual is undergoing concurrent therapy with another therapeutic agent, and the age, height, weight, health, and physical condition of the individual undergoing therapy. Generally, a higher dosage of this therapeutic agent is preferred with increasing weight of the subject undergoing therapy. Agent can be administered between one day and months post stress/injury. Furthermore administration can be executed in patients suffering with established interstitial fibrosis as present in end-stage heart failure.

Alternatively, the one or more nucleic acid(s) encoding e.g. the sgRNA and/or the endonuclease, the ASOs or modified ASOs, can also be delivered in the form of RNA.

To enhance expression and reduce possible toxicity, the one or more nucleic acid(s) in the form of RNA, can be modified to include one or more modified nucleoside e.g. using pseudo-U or 5-Methyl-C. Optionally, said one or more nucleic acid(s) can be under the regulation of regulatory elements in addition to a promoter.

Any suitable promoter or enhancer may be used that results in expression of one or more nucleic acid(s) into cells. Preferably, the expression of the sgRNA will be driven by a promoter preferably positioned upstream, e.g. contiguous to and upstream, such H1 or a U6 promoter, of the sequence encoding said sgRNA. Other tissue-specific promoters can be envisioned, particularly when cardiac tissue or cardiac cells are targeted.

In one aspect, the promoter is an inducible promoter that can be turned on or off at certain stages of development of an organism or in a particular tissue. Preferably, the inducible promoter will be selected from the group comprising promoters whose activity is modified in response to heavy-metal ions, isopropyl-β-D-thiogalactoside, hormones, progesterone antagonists or antibiotics. Most preferably, the inducible promoter will be selected from the group comprising Tetracycline or doxycycline (dox)-inducible promoter.

Preferable mammalian cultured cell lines, embryonic stem (ES) cells, induced pluripotent stem cells (iPSCs) will be selected - or will be derived from - heart cells such as, e.g. cardiac fibroblasts, myocytes, endothelial cells, and vascular smooth muscle cells.

The present invention further concerns a pharmaceutical composition as described herein for use in the treatment and/or prevention of a cardiac disease. Preferably, the cardiac disease is cardiac fibrosis.

The present invention further contemplates the use of a pharmaceutical composition as described herein in the preparation of a medicament for the treatment and/or prevention of a cardiac disease of the invention.

The invention also contemplates kits for the treatment and/or prevention of a cardiac disease. In one aspect of the invention, the kit comprises i) a first gene delivery vector comprising an endonuclease of the invention, and ii) a second gene delivery vector comprising at least one single guide RNA (sgRNA), or crRNA and tracrRNA, recognizing a target sequence comprising 16 to 25 nucleotides. Alternatively, the endonuclease of the invention and the at least one single guide RNA (sgRNA), or crRNA and tracrRNA, recognizing a target sequence comprising 16 to 25 nucleotides of the invention are comprised in one single gene delivery vector.

In another aspect, the invention further contemplates a kit for the treatment and/or prevention of a cardiac disease comprising a pharmaceutical composition comprises a nucleic acid (e.g. an ASO or a modified ASO) targeting an lncRNA associated with cardiac-specific super-enhancers (SEs).

In a further aspect, the invention contemplates is a kit for the treatment and/or prevention of a cardiac disease comprising a cell of the invention.

The kits of the invention may also comprise a container and a label or package insert on or associated with the container. Suitable containers include, for example, bottles, vials, syringes, etc. The containers may be formed from a variety of materials such as glass or plastic. The container holds a composition which is effective for treating the disease of disorder of the invention and may have a sterile access port (for example the container may be an intravenous solution bag or a vial having a stopper pierceable by a hypodermic injection needle). Alternatively, or additionally, the kits may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The label or package insert may comprise instructions for use thereof. Instructions included may be affixed to packaging material or may be included as a package insert. While the instructions are typically written or printed materials they are not limited to such. Any medium capable of storing such instructions and communicating them to an end user is contemplated by this disclosure.

The present invention further contemplates a method for controlling cardiac fibrosis and/or heart remodeling in a subject, said method comprising modulating the expression of one or more lncRNA associated with cardiac-specific super-enhancers (SEs) as disclosed herein.

The present invention further contemplates a method of treating and/or preventing a cardiac disease comprising modifying, a target sequence comprising 16 to 25 nucleotides of interest in a single cell or a population of cells, and reintroducing the modified single cell or population of cells into the patient in need thereof.

Preferably, a biopsy or other tissue or biological fluid sample comprising the single cell or the population of cells (e.g. an embryo) may be necessary. Stem cells such as ES cells or pluripotent stem cell that can be generated directly from adult cells, such as iPSCs, are particularly preferred in this regard. Usually, the cell is mammalian cultured cell lines, embryonic stem (ES) cells, and/or induced pluripotent stem cells (iPSCs) and will be selected - or will be derived from - heart cells such as, e.g. cardiac fibroblasts, myocytes, endothelial cells, and vascular smooth muscle cells.

The modified single cell or population of cells is/are then reintroduced into the patient in need thereof by any route of administration and/or delivery methods known in the art as described below.

A further aspect of the invention contemplates a cell or a population of cells comprising one or more nucleic acid(s) encoding i) an sgRNA), or crRNA and tracrRNA, of the invention1, or ii) an nucleic acid encoding an miRNA, siRNA, piRNA, hnRNA, snRNA, esiRNA, shRNA, or antisense oligonucleotide of the invention, or iii) a gene delivery vector of the invention.

Further contemplated in the present invention are nucleic acids encoding
v) a single guide RNA (sgRNA), or crRNA and tracrRNA, targeting a regulatory sequence of one or more lncRNA associated with cardiac-specific super-enhancers (SEs), or
vi) a single guide RNA (sgRNA), or crRNA and tracrRNA, targeting a genomic DNA sequence encoding an lncRNA associated with cardiac-specific super-enhancers (SEs), or
vii) an miRNA, siRNA, piRNA, hnRNA, snRNA, esiRNA, shRNA, or antisense oligonucleotide targeting a regulatory sequence of one or more lncRNA associated with cardiac-specific super-enhancers (SEs),
viii) or an miRNA, siRNA, piRNA, hnRNA, snRNA, esiRNA, shRNA, or antisense oligonucleotide targeting a genomic DNA sequence encoding an lncRNA associated with cardiac-specific super-enhancers (SEs).

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein. Various references are cited throughout this Specification, each of which is incorporated herein by reference in its entirety. The foregoing description will be more fully understood with reference to the following Examples.

### EXAMPLES

Examples are exemplary of methods of practicing the present invention and are not intended to limit the scope of the invention.

### Example 1

### Materials and Methods

### Primary cell culture and transfection

*Neonatal mouse CM and fibroblast isolation* - Neonatal C57B6 mice were sacrificed within the first 24h after birth and beating hearts were collected. Atria and great vessels were carefully dissected away and placed on ice in ADS buffer (H₂O, NaCl 116 mM, HEPES 20 mM, NaH₂PO₄ 1 mM, KCl 5.4 mM, MgSO₄ 0.8 mM, glucose 5.5 mM). The hearts were minced using a sterile sharp razorblade, and placed in a 1.5 ml-tubes (5-6 hearts per tube) containing 1 ml of PIB digestion buffer (ADS buffer; 0.05mg/ml collagenase type II (Worthington); 1mg/ml pancreatin; Sigma). Tissues were incubated at 37°C with shaking for 15 min. Supernatants were then collected in tubes containing complete medium (DMEM 75%, M199 25% ml, penicillin/streptavidin 1x, L-glutamine 1x, horse serum 10%, fetal cow serum 5%; Gibco). PIB buffer was added to undigested tissue fragments and digestion was repeated twice. Cells were collected by centrifugation at 800 rpm for 10 min at room temperature (RT). Pellet was then resuspended in an adequate volume of complete medium (2 ml each 5 hearts). Cells were then plated in 10 cm dishes for 45 min at 37°C, 10% CO₂ (pre-plating 1); after this step the non- myocytes adhere and the CMs remain in suspension. Supernatant was transferred to a new 10 cm dish and pre-plating step was repeated (pre-plating 2). After the second pre-plating the supernatant was collected in a new tube, CMs were counted and seeded on gelatin coated 3.5 cm plates (3x10⁵ cells per dish). The non-myocytes fraction was cultured in fibroblast medium (DMEM, fetal cow serum 10%, penicillin/streptavidin 1x) and seeded into 10 cm dishes. Cells were split using trypsin to minimize CMs contamination. Confluence was maintained below the 85% and medium was changed every second day.

*Adult mouse cardiac fibroblast isolation-* 12-week old C57/BL6 were injected with 100 U of heparin (Liquerin) 30 min before being sacrificed to avoid blood coagulation in the heart. Beating hearts were removed and washed in cold PBS. Then, hearts were canulated through the aorta and the vessel was tied using a surgical rope. Using a 1 ml syringe, HBSS solution (hanks balanced salt solution, GIBCO) was pumped in the heart through the aorta in order to wash out the blood. In the same way, 1 ml of digesting solution (HBSS; 2 mg/ml Collagenase type II (Worthington); 0.05 mg/ml Protease XIV; Sigma)) was pumped in the heart followed by 5 min of digestion at 37°C. This step was performed 3 times each heart.

After the third digestion, atria and big vessels were removed and the remaining ventricles were minced and resuspended in complete medium (DMEM, penicillin/streptavidin 1x, fetal cow serum 10%). The solution was pipetted up and down 10-20 times to reach a single cell suspension and the supernatant was collected avoiding undigested pieces. Cells were collected by centrifugation at 800 rpm for 10 min at RT. The pellet was resuspended in adequate volume of complete medium and cells were plated in 10 cm dishes overnight (pre-plating 1). After this step, the non-myocytes adhere and the CMs remain in suspension. Supernatant was transferred to a new 10 cm dish and pre-plating step repeated for another 24 h (pre-plating 2). Fresh complete medium was added to the pre-plating dishes to culture the non-myocytes cells and the supernatant was discarded. Cells were split to maintain a confluence not over the 85% and medium was changed every two days.

*Mouse lung and tail fibroblast isolation-* The lungs and the tail tips were collected from 12 weeks old C57/BL6 and washed in HBSS (Gibco). They were both minced using a sterile and sharp razorblade, and placed in a 2 ml tubes (2 lungs per tube or 5 tails per tube) containing 1 ml digestion buffer (HBSS + 10 mg/ml Collagenase type II, Worthington). Cells were incubated at 37°C with shaking for 40 min. After digestion fibroblast medium (DMEM, Fetal Cow Serum 10%, Penicillin/streptavidin 1x) was added and cells were collected by centrifugation at 800 rpm for 10 min at RT. The supernatant was discarded. The cellular pellet was resuspended in proper volume of fibroblast medium and then filtered into a 70um strainer to remove undigested pieces. Cells were counted and seeded in 10 cm dishes. Cells were split to maintain a confluence not over 85% and medium was changed every two days.

*Human fibroblasts-* Adult human dermal fibroblasts were purchased (Gibco). Primary cell cultures of human CFs were obtained by mechanical tissue enzymatic digestion with collagenases (Roche) and explant outgrowth from atrial samples obtained as discarded surgical tissue. Cultures of primary CFs were shown to express specific fibroblasts markers (i.e.vimentin) and to respond to TGF-β (1 ng/mL) stimulation. Cells were plated at a density of 5000 cells/cm² and maintained in a fibroblast medium (low glucose DMEM (Gibco) with fetal bovine serum (10%; Sigma), fibroblast growth factor-2 (FGF-2; 5.8 µg/µL; Peprotech) glutamine (2.5 mM; Gibco) penicillin/streptomycin (1%; Gibco), Fungizone (0.1%; Gibco) and HEPES (1.5%; Lonza). Medium was changed every 48 hours. Once cells reached a 60-70% confluence cells were starved in medium without fetal bovine serum (and FGF-2 0.285 µg/µL) for 2, 4, 8, 14, 24 or 40 hours. Subsequently, cells were collected for further analysis. Human dermal fibroblasts were used in passages between 3 and 6 and human CFs in passages 2 to 4. Between 6 and 10 independent samples were analyzed for each condition.

*GapmeR transfection in mouse primary cell-* Adult CFs, neonatal CFs and lung fibroblasts (3.5x10⁵ cells per 3.5 cm dish; passage 2) and CMs (1x10⁶ cells per 3.5 cm dish) were cultured overnight. The next day, medium was changed two hours before transfection. Then, a final concentration of 10 nM (if not differently specified in the text) of LNA lncRNA GapmeRs (GapmeR-*Wisper* or GapmeR-negative control A or GapmeR-Wisp2) (Exiqon) was transfected on cells using Xtreme gene HP DNA transfection reagent (Promega). After 48 hours, medium was changed and cells were collected for future experiments. Total RNA was obtained using miRNeasy kit (Qiagen) and the knock down confirmed by qRT-PCR.

*GapmeR transfection in human CFs-* Transfection was performed in 25 cm² flasks in cells at 80% confluence. A final concentration of 25 nM of LNA lncRNA GapmeRs (GapmeR-*Wisper* or GapmeR-negative control A; Exiqon) was transfected on cells using Xtreme gene HP DNA transfection reagent (Promega) in complete medium. After 24 hours, cells were kept in normal medium or exposed to serum starvation for another 24 hours, and subsequently were collected for further studies. Three independent samples were analyzed for each condition.

### Animal experiments - Tissue collection and preparation

Mice were sacrificed by deep anesthesia followed by cervical dislocation, and organs were collected. Hearts were dissected from sham and MI mice at different time points after artery ligation. Atria and big vessels were eliminated. The remaining ventricles were rinsed in diethylpyrocarbonate (DEPC)-treated PBS to minimize residual blood contamination. Furthermore, the ventricles were divided in 3 parts: top section (from the top to the ligature), central section (from the ligature to the center of the infarcted area) and the tip of the heart (from the center of the infarcted area to the bottom). The heart tip (50% viable muscle, 50% infarcted zone) was used to collect total RNA. The central section was fixed in formalin and embedded in paraffin. The top section was snap frozen in liquid nitrogen and stored at -80'C for future analysis.

### RNA isolation, cDNA preparation and quantitative PCR analysis

Total RNA from tissues and cultured cells isolated from mice was extracted using miRNeasy kit (Qiagen) according to the manufacturer's instructions and quantified with Nanodrop instrument. The quality control was performed with a Agilent 2100 bioanalyzer (Agilent Technologies). Two steps cDNA synthesis was performed with SuperScript II (Invitrogen), and quantification was carried out using QuantStudio 6/7 (Thermofisher). Gene expression was normalized to *Gapdh* and quantified using the ΔΔCt method.

Total RNA from human cells was obtained using the Maxwell 16 LEV simplyRNA Purification Kit (Promega) according to manufacturer's instructions, and was quantified with a Nanodrop instrument. Reverse transcription was performed using the high capacity cDNA reverse transcription kit (Applied Biosystems). Real-time PCR for collagen-related genes was performed with a StepOne Plus Real-time PCR system according to the manufacturer's recommendations (Applied Biosystems) using specific TaqMan fluorescent probes. Data were normalized to ribosomal 18S expression. *WISPER* and *WISP2* expression were analyzed with a 7900HT Fast Real-Time PCR system (Applied Biosystems) using the power SYBR green PCR master mix (Applied Biosystems) and specific primers. Gene expression was normalized to *GAPDH.*

### Western blotting

Proteins were extracted from neonatal CFs transfected with *GapmeR-Wisper* (10 nM) or GapmeR-Scrambled (10 nM) using lysis buffer (50 mM Tris-HCl, 150 mM NaCl, 0.25 % DOC, 1% Nonidet P-40, 1% Triton X-100 containing protease and phosphatase inhibitors (Roche)). Proteins were resuspended in SDS-PAGE buffer, separated on acrylamide gels and transferred to PVDF membranes (BioRad). Membranes were incubated with mouse monoclonal anti-α-SMA primary antibodies (Sigma, 1:2000 dilution), and then with horseradish-conjugated secondary antibodies (Amersham Bioscience, 1:2000 dilution). α-SMA abundance was quantified by densitometry and normalized to the total amount of proteins.

### Thymidine incorporation assay

Adult CFs and lung fibroblasts were transfected with 10 nM of GapmeR-*Wisper* or GapmeR-Scrambled for 24h. 4x10³ cells/well were seeded in 96 round bottom-well plates in quadruplicate. 1 µCi of [H³]-Thymidine was added in each well at 0h, 24h and 48h after cell seeding, and incubated for 18 h at 37°C. Cells were then harvested and transferred into a filter plate. Radioactivity was measured using a scintillation beta-counter (TopCount, Canberra Packard). Measurements were performed in quadruplicate for each condition in 5 independent experiments and normalized to 0 h values.

### Wound closure assay

Adult CFs and lung fibroblasts were transfected with 10 nM of *GapmeR-Wisper* or GapmeR-Scrambled for 48h. 3.5x10⁵ cells were seeded in 3.5-cm well and let them attached overnight. The scratch test was performed using a sterile 10 µl filter tip. Medium was replaced twice to remove floating cells. Three pictures for each condition were taken at 20x magnification every 24 hours. The size of the wound has been measured using ImageJ. Measurements were performed in triplicate for each condition in at least 3 independent experiments and normalized to 0 h values (100%).

### Annexin V-positive cells quantification

Adult CFs and lung fibroblasts were transfected with 10 nM of GapmeR-Wisper or GapmeR-Scrambled. Transfected cells were collected after 48h and resuspended in FACS medium. Cells were stained using the FITC Annexin V Apoptosis detection Kit (BD Pharmingen) according to the manufacturer's instructions. Annexin V and PI signals were quantified with a Galios FACS apparatus within 30 min after staining. Results were analyzed with FlowJo.

### Wisper in situ hybridization

Infarcted (14 days post-MI) and sham-operated hearts were fixed for 48h in 10% neutral buffered formalin and sections of 4 µm were obtained. Sections were de-waxed, dehydrated, air- dried briefly and incubated in 1.3 mg/ml Pepsin/0.1mM HCl solution for 5 min at 37°C. After a brief wash in DEPC MQ, sections were incubated overnight at 42.5°C in 1x hybridization buffer (ENZO Life sciences,) with either 100 nM *Wisper* DIG labeled probe (Exiqon) or a scrambled control probe. The following day the sections were washed once in 5xSSC and twice in 0.2xSSC at hybridization temperature. Subsequently, sections were blocked in DIG blocking buffer from the DIG Wash and Block Buffer Set (Roche) for 20 min followed by a 45 min-incubation in 1:500 anti-DIG-AP, Fab fragments (Roche). Sections were washed 3x5 min with TBS and incubated 3 min in DIG detection buffer at RT. Alkaline phosphatase signal was detected by using NBT/BCIP tablets (Roche) for 4 hours at 30°C in darkness. Finally, sections were counterstained with fast red (Sigma) briefly washed in MQ, quickly dehydrated through an ethanol gradient and mounted with entellan (EMS). Pictures were taken using a Nikon Stereomicroscope SMZ 25 at different magnifications.

### BrdU labeling and detection

Mice were supplied with a solution of 10 mg/ml 5-Bromo-2-deoxyuridine (BrdU; Sigma) supplemented with 1% glucose directly in the drinking water for 7 days. The drinking solution was changed every second day. For BrdU detection, frozen heart tissue sections were fixed in 2% paraformaldehyde (PFA) in PBS for 20 min at room temperature. DNA was fragmented by incubation in 2N HCl at 37°C for 20 min and neutralization in 0.1M Borate buffer, pH 8.5. BrdU detection was performed using rat anti-BrdU antibody (Abcam; 1: 100) and Alexa-Fluor 594 conjugated anti-rat antibody (Molecular Probes; 1:250). For BrdU-laminin co-immunostaining, tissue sections were first subjected to immunofluorescence staining to detect protein antigens. Tissue sections were post-fixed with 2% PFA for 10 min at room temperature, and then processed for BrdU detection.

### Study design

The main goal of our study was to evaluate cell-specific lncRNAs as therapeutic targets for cardiac fibrosis and heart disease. Using a stringent pipeline, we selected high priority lncRNA candidates that are associated with cardiac-specific SEs. We focused our attention on one cardiac fibroblast (CF)-enriched lncRNA named *Wisper.* Loss of function experiments were performed to assess the role of *Wisper* in the biology of CFs and in fibrosis. Modified ASOs (GapmeRs) were used to silence *Wisper* expression in mouse CFs. Knockdown experiments were performed in human CFs to verify the evolutionary conserved function of this transcript. For experiments in vivo, LAD artery ligation was chosen as a well-established animal model of myocardial infarction. Experimental groups include at least 6 animals to robustly identify alteration in cardiac function and fibrosis. Mice were randomly assigned to treatment groups. To test if *Wisper* may hold therapeutic potential in cardiac fibrosis, we performed loss of function experiments in vivo via GapmeR injection. In the preventive approach, injection was performed three days before myocardial infarction. In the therapeutic approach, GapmeRs were injected 2 and 9 days after infarction. GapmeR injection and echocardiographic measurements were double blinded until statistical analysis. Cardiac fibrosis was quantified by Masson's trichrome staining on heart sections. All sample measurements were blinded. In addition, to evaluate the tissue specificity of *Wisper* expression, we took advantage of the one-kidney one-clip (1K1C) model of renovascular hypertension in the mouse. This model is characterized by the development of cardiac hypertrophy and fibrosis secondary to volume-dependent hypertension. Fibrosis also develops in the clipped kidney, allowing direct comparison of *Wisper* expression in two different fibrotic organs. *WISPER* human ortholog was identified and detected in RNA isolated from human cardiac biopsies of patients suffering from aortic stenosis. This pathology is associated with extensive myocardial fibrosis that directly contributes to left ventricle dysfunction and heart failure. Samples of cardiac tissue from AOS patients (n=26) were divided in two groups after analyzing the distribution for collagen volume fraction as described in the text. No data were excluded in this analysis.

### RNA-Seq based IncRNA profiling after myocardial infarction

RNA-Seq on infarcted and sham-operated hearts (14 days after infarction), *ab initio* transcript reconstruction, differential expression analysis of lncRNAs, heart-specificity analysis and human ortholog identification datasets were previously described (29).

### Super-enhancer mapping to human IncRNA orthologs

The locations of super-enhancers were downloaded from (12). These regions were defined using H3K27ac ChIP-Seq from the Epigenome Roadmap (56). LncRNAs arising from super-enhancers and typical enhancers were determined by genomic overlap.

### Animal experiments

Animal experiments were approved by the Government Veterinary Office (Lausanne, Switzerland) and performed according to the University of Lausanne Medical School institutional guidelines. *Myocardial infarction model -* Myocardial infarction in mice (numbers of animals per group are in the figures legends) was induced as previously described (57). Briefly, male C57/BL6 (Charles River) at 12 weeks of age were anesthetized by i.p. injection of ketamin/xylazine/acepromazin (65/15/2 mg/kg), and placed on artificial ventilation. After left thoracotomy, the pericardium was gently opened and a 7.0 silk ligature (Aesculap) was tied around the LAD artery near the insertion of the left auricular appendage. Occlusion of the artery was verified by the rapid blanching of the left ventricle. In sham-operated mice, the ligature was placed in an identical location but not tied. After surgery, mice were gradually weaned from the respirator until spontaneous respiration was resumed and replaced in the cage.

*One-kidney one-clip renovascular hypertension in mice* - One-kidney one-clip (1K1C) renovascular hypertension in mice was produced as previously described (35). Briefly, a left lateral abdominal incision is used to expose the kidney. A clip (0.12 mm-opening) is placed around the left renal artery in order to reduce renal blood flow. Another incision is made in the right lateral abdominal wall and a right nephrectomy is performed. Animals develop volume overload-dependent cardiac hypertrophy and renal failure.

*Echocardiography -* Transthoracic echocardiography was performed using a 30-MHz probe and the Vevo 770 Ultrasound machine (VisualSonics). Mice were lightly anesthetized with 1% isoflurane, maintaining heart rate at 400-500 beats per minute, and placed in dorsal recumbency on a heated 37°C platform. The heart was imaged in the 2D mode in the parasternal long-axis view. From this view, an M-mode curser was positioned perpendicular to the interventricular septum and the posterior wall of the left ventricle (LV) at the level of the papillary muscles. LV free wall thickness in diastole (LVWT d) and in systole (LVWT s) as well as LV diameter in diastole (LVD d) and in systole (LVD s) were measured according to the American Society of Echocardiography guidelines. The measurements were taken in 3 separate M mode images and averaged. Ejection fraction (EF) was calculated using the formula % EF = [(LVVD - LVVS) / LVVD] X 100, where LVVD and LVVS are LV volume in diastole and systole respectively.

*GapmeR delivery in vivo* - GapmeR-*Wisper* and GapmeR-Scrambled control A (Exiqon) were diluted in NaCl 0.9% isotonic solution to obtain the final exact concentration (5, 10 or 15 mg/kg) just before the injection. GapmeRs were delivered i.p. using a U100 insulin 0.3 ml syringe and a 30GX8 mm needle.

### Primary cell culture and transfection (as described above)

*Neonatal mouse CM and fibroblasts* - Cells were obtained from neonatal C57/BL6 mice.

*Adult mouse cardiac*, *lung and tail fibroblasts -* Cells were obtained from 12-week old C57/BL6 mice.

*Human fibroblasts-* Adult human dermal fibroblasts were purchased from Gibco. Primary cell cultures of human CFs were obtained by tissue enzymatic digestion with collagenase and explant outgrowth from atrial samples as previously described (58).

*GapmeR transfection in mouse primary cell-* Adult CFs, neonatal CF, lung fibroblasts and CMs were transfected with 10 nM (if not differently specified in the text) of LNA lncRNA GapmeRs (GapmeR-*Wisper* SEQ ID No. X or scrambled GapmeR SEQ ID No. X or GapmeR-Wisp2 SEQ ID No. X; Exiqon).

*GapmeR transfection in human CFs-* Fibroblasts were transfected with 25 nM of LNA lncRNA GapmeRs (GapmeR-*Wisper* or scrambled GapmeR; Exiqon).

### CRISPR-on assay

CRISPR-based gain-of-function was used to activate *Wisper* expression in P19CL6 cells (RCB2318, RIKEN Cell Bank, Japan). Cells were cultured in DMEM with 10% FCS and transfected with component of the synergistic activation mediator described by the Zhang laboratory in combination of a Wisper-targeting guide RNA engineered to contain two MS2 aptamers (sgRNAMS2; Addgene plasmid #61424) and containing the following sequence: GTCGACTCTGCTATACTCCA (SEQ ID No. 13). Plasmids were transfected at a 1:1 ratio using Lipofectamine 2000 (Life Technologies) according to manufacturer's instructions. Total RNA was isolated 48 h after transfection using miRNeasy kit (Qiagen) and subjected to qRT-PCR.

### Cytoplasmic and nuclear compartment fractionation

2x10⁶ adult CFs were washed twice with cold PBS. Nuclear and cytoplasmic RNA fractions were isolated using the Cytoplasmic and Nuclear RNA purification kit (Norgen Biotek Corp) according to the manufacturer's instructions. Total RNA was isolated from 1x10⁶ adult CFs using miRNeasy kit (Qiagen) and used as input.

### Immunohistochemistry

Cardiomyocytes - CMs were transfected with 10 nM of GapmeR-*Wisper* or GapmeR-Scrambled for 48h. Cells were then fixed for 10 min in 4% paraformaldehyde in PBS and permeabilized with 0.2% Triton X100 in PBS. After treatment with blocking buffer (PBS containing 0.001% Triton X100, 1% BSA and 1% FCS), cells were incubated overnight at 4 °C with anti α-sarcomeric actinin antibodies (1:400; Sigma). One day after, cells were washed 3 times and incubated 1h at RT in the dark with conjugated anti-mouse donkey secondary antibodies (1:500; Alexa Fluor 488, Life Technology). Nuclei were stained with DAPI (Invitrogen). Unbound antibodies were washed out with PBS 0.1% Tween. Subsequently, coverslips were mounted with Fluoromount-G (Southern Biotech) and analyzed with an inverted Axiovision Observer Z1 fluorescence microscope (Carl Zeiss). Five pictures for each different condition were taken. The dimension of the cells was measured using ImageJ. CMs were counted as α-sarcomeric actinin-positive and DAPI positive cells while non-myocyte cells were counted as α-sarcomeric actinin-negative and DAPI positive cells.

Cardiac Fibroblasts - Cells were transfected with 5 nM of GapmeR-*Wisper* or GapmeR-Scrambled for 48h. Cells were fixed as described above and incubated overnight at 4 °C with anti-TIAL antibody (1:200; Abcam, Ab129499). One day thereafter, cells were washed 3 times and incubated 1h at RT with conjugated anti-rabbit goat secondary antibodies (1:250; Alexa Fluor 488, Life Technology). Actin filaments were stained using Texas Red-X Phalloidin (1: 100; Life Technologies; T7471) and nuclei were stained with DAPI (Invitrogen). Unbound antibodies were washed out with PBS 0.1% Tween. Subsequently, coverslips were mounted with Fluoromount-G (Southern Biotech) and TIAL localization was analyzed with an inverted Axiovision Observer Z1 fluorescence microscope (Carl Zeiss).

### Assessment of cardiac fibrosis

Hearts were harvested from sham-operated and MI mice, and atria and big vessels were eliminated. Cardiac tissues were fixed in 4 % formalin overnight. Paraffin tissue sections were processed for Masson's trichrome staining using standard histological procedures. The percentage of fibrotic tissue was determined by measuring collagen deposition (blue) on Masson's trichrome-stained sections using ImageJ.

### RNA pulldown

Biotinylated *Wisper* sense and *Wisper* antisense were in vitro transcribed using the T7 or T3 RNA polymerase (Promega) and Biotin RNA Labeling Mix (Roche), then purified with Quick Spin columns (Roche) according to manufacturers' instructions. 4µg of biotinylated RNA was denaturated for 5 min at 65°C in RNA Structure Buffer (10 mM Tris-HCl, 10mM MgCl₂, 100 mM NH₄Cl) and cooled to RT. In brief, freshly harvested cardiac fibroblasts were washed in ice-cold PBS. Cells were lysed in 1 ml Lysis Buffer (25 mM Tris-HCl pH 7.4, 150 mM NaCl, 5 mM MgCl₂, 0.1 mM EDTA, 0.5% NP40, 1mM DTT, 1mM PMSF, 0.1 U/µl RNase inhibitor (promega) and 1x protease inhibitor cocktail (Sigma)). Streptavidin Dynabeads were washed in NT2 Buffer (50 mM Tris-HCl pH 7.4, 150 mM NaCl, 1 mM MgCl₂, 0.05% NP40, 1mM DTT, 20 mM EDTA, 400 mM Vanadylribonucleoside, 0.1 U/µl RNase inhibitor (Promega) and 1x protease inhibitor cocktail (Sigma)) and used to preclear the lysate (20 min at 4°C). The pre-cleared cell lysate was incubated with biotinylated RNA along with 0.1 U/µl RNase inhibitor (Promega) and 20 µg/ml Yeast tRNA (ambion) for 1.5 h at room temperature followed by addition of 60 µl of Streptavidin Dynabeads for 1.5 h. The beads containing the RNA protein complex were washed thrice in NT2 Buffer then were directly boiled in SDS-gel loading dye. Retrieved proteins were loaded on a 12% polyacrylamid gel in denaturating SDS-PAGE buffer and visualized with Candiano colloidal Coomassie staining. Mass spectrometry were performed at the PAF (Protein Analysis Facility, University of Lausanne, Switzerland). Analysis of the pulled-down proteins was performed using Scaffold4. Proteins enrichment was calculated by Fisher's exact test.

### Western Blot of RNA pulldown

4% of the samples used for RNA pulldown were utilized as input. Proteins were resolved on 10% SDS-PAGE mini-gels and transferred to PVDF membranes (BioRad). The membrane were blocked and incubated with primary antibody against TIAR (Santa Cruz Biotechnology, Inc) at 4°C overnight. The primary antibody was detected using infrared IrDye antibody regents and scanning the membranes using Odyssey Infrared Imaging System (LiCor Biosciences). Protein quantification on the scanned Western blots was performed using the software provided with the scanner (LiCor Biosciences). 20 µg of mouse TIAR Lysate (Santa Cruz Biotechnology) was used as a control.

### RNA immune precipitation (RIP)

Cells were harvested in Lysis Buffer as described previously. Supernatant was pre-cleared with Dynabeads G (Invitrogen). The pre-cleared lysate was incubated with normal IgGor with anti-TIAR antibody (Santa Cruz Biotechnology) at 4°C overnight followed by addition of 50 ul of Dynabeads G for 1h at 4°C. The immunoprecipitated complexes was washed thrice in NT2 Buffer and eluted in 500 µl of Quiazol. Total RNA extraction and cDNA synthesis were performed as described before. Real-Time PCR system (Applied Biosystems) was then used to measure expression of *Wisper*, *Wisp2* and *Plod2.*

### Human tissue sampling

The present study is conformed to the principles of the Declaration of Helsinki. All subjects were duly informed and gave written consent. Samples were collected as previously described (*46*). *Collagen volume fraction (CVF)* - The fraction of myocardium occupied by collagen was quantified as previously described (*46*). A cluster analysis was performed according to the CVF values to define the non-severe (CVF < 12%; n = 11) and the severe (CVF > 12%; n = 15) fibrosis groups (*46*). *Echocardiographic analysis-* LV mass was measured from M-mode recordings LV mass index (LVMI) was calculated by dividing LV mass by body surface area. LV end-systolic and end-diastolic volume indexes (LVESVI and LVEDVI, respectively), corresponding to the LV volumes corrected by body surface area, and the LVEF were determined in all patients. The following pulsed Doppler measurements were obtained: maximum early (VE) transmitral velocity in diastole, maximum late (VA) transmitral velocity in diastole, the deceleration time of the early mitral filling wave (DT), and the isovolumetric relaxation time (IVRT).

### Sequencing of RNA isolated from GapmeR-transfected adult CFs

Total RNA was isolated from adult CF transfected (10 nM, 48 h) with GapmeR-*Wisper* (n=4) or GapmeR-Scrambled (n=4) using the miRNeasy kit (Qiagen). Sequencing libraries were prepared according to Illumina RNA Seq library kit instructions with PolyA selection. Libraries were sequenced with the Illumina HiSeq2000 (1x100 bp). Purity-filtered reads were adapter and quality trimmed with Cutadapt (v. 1.3), and filtered for low complexity with seq_crumbs (v. 0.1.8). Reads were aligned against the Mus musculus.GRCm38.82 genome using STAR (*59*) (v. 2.4.2a). The number of read counts per gene locus was summarized with htseq-count (*60*) (v. 0.6.1) using Mus musculus.GRCm38.82 gene annotation. Quality of the RNA-Seq data alignment was assessed using RSeQC (*61)* (v. 2.3.7). Reads were also aligned to the Mus musculus.GRCm38.82 transcriptome using STAR (*59*) (v. 2.4.2a), and the estimation of the isoform abundance was computed using RSEM (*62*) (v. 1.2.19). Statistical analysis was performed for genes and isoforms independently in R (R version 3.1.2). Genes/Isoforms with low counts were filtered out according to the rule of 1 count per million (cpm) in at least 1 sample. Library sizes were scaled using TMM normalization (*63*) (EdgeR v 3.8.5) and log-transformed with limma voom function (R version 3.22.4). Statistical quality controls were performed through pairwise sample correlations, clustering and sample PCA. Replicates cluster together and are well separated between conditions. Differential expression was computed with limma (*64*) by fitting data into a linear model, adding the factor for the batch effect and comparing GapmeR versus control conditions. The *P*-values were adjusted for multiple comparisons using the Benjamini-Hochberg method (*65*), controlling for false discovery rate (FDR) or adjusted *P* value.

### Statistical analysis

GraphPad Software (version 6 or 7) was used for statistical analysis. Data throughout the paper are expressed as mean ± SEM. Statistical significance between two columns was assessed by two-tailed unpaired Student's t test; for more than two columns, one-way ANOVA (Analysis of variance; Fisher's LSD test) analysis was used. Two-way ANOVA (Fisher's LSD test) was used to evaluate statistical significance between two or more groups. Correlation analysis was performed with Pearson (R or R² values; 95% confidence interval) or Spearman (R; 95% confidence interval) test. Significance in percentage of animal survival was calculated with Log-rank (Mantel-Cox) test. *P* values < 0.05 were considered significant in all events.

### Results

### Wisper is cardiac SE-associated lncRNAs

Emerging evidence suggests that SEs and the lncRNAs associated with them represent specific regulators of cell state and identity during development and disease (*10*). Based on this cogent rationale, we set out to identify SE-associated lncRNAs modulated in the damaged myocardium, which were conserved among mouse and human genomes (*66*). Previously, our laboratory identified 1521 novel lncRNAs in the murine heart (*29*). A large number of these novel lncRNAs were associated with active cardiac-specific enhancers. Using this transcriptomic dataset, we first filtered for all novel lncRNAs that were classified as heart-enriched. To facilitate downstream functional assessment and alleviate any confounding interpretations based on overlapping protein coding genes (PCGs), we further filtered lncRNAs for those that were intergenic and differentially expressed in the border zone (BZ) 14 days post-MI, resulting in the inclusion of 149 lncRNAs (*66*))*.* To identify putative human orthologs, these transcripts were mapped to the human genome using TransMap, a cross-species alignment tool. Globally, of these 149 mouse lncRNAs, 130 were predicted to have human orthologs. Considering that lncRNAs associated with TEs and SEs likely represent high priority functional candidates (*10*), we next examined an enhancer catalogue generated in 23 human tissues that utilized histone 3 lysine 27 acetylation (H3K27Ac) marks and the ROSE algorithm to identify tissue-specific TEs and SEs (*12*). We found that 37 (28%) of our lncRNAs map to TEs and 6 (5%) mapped to human heart-specific SEs. The most upregulated lncRNA in the infarcted mouse heart was one of the six SE-associated lncRNAs, supporting the notion that these transcripts could play important roles in the transcriptional reprogramming that underpins cardiac remodeling (*66*). To dissect the cardiac cell specificity of the six SE-associated lncRNAs, expression was evaluated in CFs and CMs isolated from the adult murine heart. Expression of these transcripts and canonical PCGs was determined via qRT-PCR (Fig. 1). The CM-specific PCGs, *Tnni3* and *Actc1*, were highly enriched in CM-preparations whereas CF-specific PCGs as *Col1a1*, *Tgfb2*, *Postn*, *Vim* were specifically enriched in CFs. Among the SE-associated lncRNAs, two were significantly enriched in CMs (*P* = 0.009 and 0.42 respectively) while one lncRNA was highly enriched in CFs (*P* < 0.001). In fact, this transcript was more enriched in CFs than the CF-specific PCGs, suggesting it could have important functions in this particular cardiac cell type and therefore could play a role in the fibrotic response of the heart after infarction. Proximal to this lncRNA was the gene encoding the matricellular protein *Ccn5* (*Wisp2*), which had recently been implicated in pathological myocardial fibrosis (*31*). We therefore named this lncRNA: WIsp2 SuPer-Enhancer associated RNA or *Wisper*, and its human ortholog *WISPER.* This transcript was found substantially conserved in the two species with a 57.3% sequence identity between the two orthologs. Importantly, the SE from which this lncRNA derived was uniquely active in the adult human heart as compared to other tissues (*66*). Nevertheless, the SE overlapped with *WISP2* sequences, suggesting that it contains several constituent enhancers, which may encode different *cis*-regulatory potential (32). Considering the enrichment of this transcript in CFs, coupled to its upregulation in the BZ after MI, we suspected it could represent an interesting target molecule for the regulation of pathological fibrosis and was selected for further investigation.

### Wisper expression is enriched in cardiac fibroblasts and associated with cardiac fibrosis

Integrative transcriptome analysis using publicly available RNA-Seq datasets demonstrated that *Wisper* was a heart-enriched transcript (*29*). To validate this finding, qRT-PCR was conducted using RNA isolated from different adult mouse tissues. *Wisper* was more expressed in the heart than all other tissues (*66*). On the other hand, *Wips2* expression was not characterized by the same tissue distribution. We next quantified *Wisper* expression in fibroblasts of cardiac and non-cardiac origins. In comparison to fibroblast-associated PCGs such as *Tgfb2*, *Fn1*, *Col3a1* and *Col1a1*, which were similarly expressed in fibroblasts from different sources, *Wisper* was significantly enriched in fibroblasts isolated from neonatal and adult hearts (Fig. 2A; *P* < 0.001). Importantly, several binding sites for cardiac TFs such as GATA4 and NKX2-5 were identified in the SE element from which *WISPER* derives (*66*). This was in contrast to *WISP2,* which was characterized by distinct TF binding sites at its promoter such as ATOH1 binding sites and E-box that were known to regulate lung-specific expression (*66*) (*33*). Finally, lncRNA functions are typically dependent on subcellular localization, with those present primarily in the nucleus involved in chromatin regulation whereas cytoplasmic lncRNAs influence post-transcriptional processes (*24*). *Wisper* was found equally distributed between the two subcellular compartments, indicating it could play roles in both transcriptional and post-transcriptional regulatory processes (Fig. 2B).

After MI, the myocardium undergoes a remodeling process that is characterized by three distinct phases. These include an inflammatory response (day 1-3 post injury), proliferation and granulation tissue formation (day 7-14), and scar maturation (day 21-28) (*34*). Pro-fibrotic pathways are typically associated with the proliferative phase, in which differentiation of myofibroblasts and subsequent proliferation, migration and secretion of ECM components take place. In order to assign *Wisper* to either of these phases, MI was induced in the mouse heart by ligation of the left anterior descending (LAD) artery and *Wisper* expression was assessed over a 28-day period. Cardiac dimensions and function were assessed by echocardiography (Fig. 2C; Table 1). Gene expression profiling demonstrated the expected expression kinetics for fibrotic and hypertrophy-associated PCGs (Fig. 2D). Importantly, *Wisper* was maximally expressed 14 days post-MI, corresponding to the proliferative phase in which myofibroblasts migrate to the BZ and actively secrete collagen and other ECM components. The temporal kinetics of *Wisper* induction implicated this transcript in cardiac fibrosis driving pathological remodeling. To support these findings, RNA *in situ* hybridization using probes against *Wisper* was performed on mouse hearts 14 days after infarction. In accordance with *Wisper* expression in activated fibroblasts, a marked signal was observed in the BZ of infarcted hearts (*66*). *Wisper* expression was also detected, albeit less frequently, in the interstitial space of the viable muscle. We therefore evaluated whether *Wisper* expression correlated with gene programs linked to cardiac fibrosis. *Wisper* expression was highly correlated with PCGs relevant to ECM deposition (*66*), and also highly correlated with echocardiographic traits linked to remodeling of the injured myocardium (*66*). Moreover, in order to evaluate the tissue specificity of *Wisper* expression under stress conditions, we used a mouse model of renovascular hypertension, namely the one kidney-one clip model (*35*). Cardiac hypertrophy and fibrosis develop in response to volume overload in this model (Fig. 9A-C). In addition, the single hypoxic kidney is also characterized by extensive fibrosis (Fig. 9C). Strikingly, *Wisper* was induced in the stressed heart but not in the stressed kidney. In contrast, *Wisp2* expression was upregulated in both organs.

### Wisper controls cardiac fibroblast behavior and survival

In order to characterize the functional role of Wisper, a loss of function approach was utilized in isolated adult murine CFs. Modified antisense oligonucleotides (ASOs) called GapmeRs were used to initiate nuclear ribonuclease H-mediated degradation of the transcript and to deplete *Wisper* in both the nucleus and cytoplasm (Fig. 8A). Titration experiments showed that 10 nM of GapmeRs targeting *Wisper* was sufficient to achieve maximal knockdown in adult CFs without affecting CF integrity (Fig. 3A). This concentration was therefore used for subsequent experiments. Silencing of *Wisper* expression resulted in a specific impact on ECM-associated PCG expression (Fig. 3B). *Col3a1, Fn1* and *Tgfb2* were downregulated, however *Col1a1* and *Ctgf* were not affected. *Wisper* depletion led also to the downregulation of the proximal PCG *Wisp2,* suggestive of a possible *cis-*regulatory role in adult CFs. Considering the effect on gene expression, we suspected *Wisper* could be fundamentally involved in the transdifferentiation of CFs into myofibroblasts. We therefore isolated neonatal murine CFs, which can be induced to differentiate through cell passaging in vitro. Differentiation of these cells resulted in the upregulation of myofibroblast-specific PCGs such as *Tgfb2, Fn1, Col1a1, Col3a1* and *aSma* (*66*)*. Wisper* and *Wisp2* expression was closely associated with myofibroblast differentiation. Importantly, *Wisper* depletion in differentiated myofibroblasts resulted in a significant downregulation of *Col3a1, Fn1, Tgfb2* and *aSma* expression (Fig. S3B of (*66*); *P* < 0.001), similar to what was observed in adult CFs (Fig. 3B of (66)). The myofibroblast-associated α-SMA protein was also significantly downregulated (Fig. 10; *P =* 0.011). Again, *Col1a1* was not affected by *Wisper* depletion supporting a specific regulatory role on individual ECM genes.

As *Wisper* depletion was found to have a large impact on fibroblast identity, we proceeded to evaluate cell behavior after *Wisper* knockdown in adult CFs. We found a significant decrease in proliferation in CFs 24 hours after *Wisper* knockdown (Fig. 3C; *P* < 0.001), suggesting that *Wisper* was important for the proliferative ability of CFs. Using a wound closure assay, we demonstrated a significant attenuation of the migratory ability of *Wisper-depleted* CFs (Fig. 3D; *P* < 0.001). Finally, apoptosis was assessed in *Wisper-depleted* CFs via testing annexin V positivity by flow cytometry analysis. At 24 hours post-transfection, there was a five-fold increase in the percentage of apoptotic CFs treated with GapmeRs targeting *Wisper* (Fig. 3E). In addition, the ratio between *Bax* and *Bcl2* expression indicated an increase in pro-apoptotic signaling in CFs upon *Wisper* depletion (*66*). Altogether, these data suggest that *Wisper* knockdown impacts cell survival in CFs. To confirm the cardiac specificity of *Wisper* function, we used the same dose of *Wisper*-targeting GapmeRs in fibroblasts of non-cardiac origin, i.e. lung fibroblasts (*66*), and observed no impact on expression of fibroblast-associated PCGs (*66*), proliferation (*66*), migration (*66*) and apoptosis (Fig. 3F). Finally, despite being largely CF-enriched, *Wisper* is also expressed at low concentrations in CMs. Therefore, we also tested the effects of GapmeRs in neonatal CMs to detect any unanticipated effects on these cells (Fig. S3D of (*66*)). Although slightly decreasing *Wisper* expression, GapmeR treatment did not impact CM-specific gene expression, structure, cross-sectional area or cell number. Interestingly, the number of CFs, which are typically present in neonatal CM cultures, was reduced following Wisper depletion, suggesting that decreased *Wisper* expression in CM cultures reflects modulation in contaminating CFs.

### Wisper regulates specific cardiac fibroblast gene programs

To determine whether *Wisper* played a global role in the regulation of specific CF gene programs, RNA-Seq was performed on scrambled and *Wisper*-specific GapmeR-treated adult CFs. We identified 3153 differentially expressed protein coding genes (PCGs) (fold change >2, adjusted *P-*value <0.05) in *Wisper*-depleted CFs, 1337 upregulated and 1816 downregulated (Fig. 4A of (*66*)). Using Gene Ontology (GO) analysis, we found that upregulated PCGs were associated with biological processes linked to the control of cell cycle and mitosis (Fig. 4B). Furthermore, these PCGs were also associated with mouse phenotypes linked to abnormal control of cell cycle and induction of cell death, both processes observed in *Wisper*-depleted CFs. Downregulated PCGs were associated with modulation of the immune response and inflammatory phenotypes in the mouse (Fig. 4C of (*66*)). These findings are consistent with the important roles that CFs play during acute inflammation after infarction (*34*). Upregulated genes included many important pro-apoptotic molecules (e.g. *Dusp6* and *Casp3*) whereas anti-apoptotic genes were downregulated (e.g. *Bcl2l1* and *Bcl2a1b*) (Fig. 4D of (*66*)). Similarly, cell cycle inhibitors (e.g. *Btg1*, and *Tob1*) were induced upon *Wisper* deletion whereas cell cycle activators (e.g. *Ccnd1*) were downregulated. Many regulators of the immune response were also downregulated (e.g. *Cxcl10*). Importantly, key PCGs linked to the ECM (e.g. *Col4a6* and *Col8a1*) were depleted in *Wisper* GapmeR-treated cells. Furthermore, we also tested the capacity of *Wisper* to induce a fibroblastic gene program in non fibroblastic cells when enacted at its own site of transcription. We used a CRISPR-based gain-of-function approach (CRISPR-on). P19CL6 cells were transfected with components of the synergistic activation mediator described by the Zhang laboratory in combination with a Wisper-targeting guide RNA engineered to contain two MS2 aptamers (*36*). Significant *Wisper* expression was measured 2 days following transfection (*P* < 0.011). In turn, prototypic fibroblast genes were induced. Interestingly, *Wisp2* was not activated in these cells (Fig. 4). Considering the cis-based regulatory roles linked to SE-associated lncRNAs, we proceeded to assess the impact of *Wisper* depletion on proximal PCGs embedded within its topologically associated domain (TAD). Cell-type invariant TADs are typically established during pluripotency and are critical for configuring the three-dimensional chromatin architecture that ensures correct temporal and spatial interactions between distal enhancers and their target promoters. We therefore utilized publicly available high-throughput confirmation capture datasets from mouse embryonic stem cells to interrogate the topological nature of this locus (Fig. S4A of (*66*)). Of the ten PCGs within the *Wisper*-harboring TAD, five were differentially expressed upon *Wisper* depletion in adult CFs, and among them *Wisp2.* We therefore evaluated whether *Wisper* could exert its action via cis-regulation of *Wisp2* expression. We examined the specificity of the gene expression programs in CFs after GapmeR-mediated *Wisp2* depletion, which resulted in a significant loss of *Wisp2* expression (*P* < 0.001) without affecting *Wisper* concentrations (Fig. 4F of (66)). The canonical ECM proteins previously examined, whose expression was modified by *Wisper* depletion, were not impacted by *Wisp2* knockdown. These data support a role for *Wisper* in dictating gene programs associated with cell identity and behavior in CFs, independent of *Wisp2* expression. Finally, the RNA-Seq data also allowed us to assess the impact of *Wisper* depletion on the annotated long noncoding transcriptome (Fig. S4B of (66)). Among the 435 upregulated lncRNAs, well-characterized lncRNAs such as *Neat1* and *Gas5* were included (Fig. S4C of (66)). Conversely, 276 lncRNAs were downregulated; among them, *Malat1*, *Ftx* and *Firre* have all been implicated in cell cycle control and apoptosis in various cell types (*37*, *38*).

### Wisper is associated with TIA1-related protein, and regulates Lysyl hydroxylase 2 expression

Many lncRNAs exert their function via interaction with proteins. In order to identify relevant *Wisper*-binding proteins, we performed a lncRNA pulldown assay. A biotinylated *Wisper* probe was therefore used as a bait to selectively extract putative *Wisper* protein partners from an adult cardiac fibroblast lysate (Fig. S4D and E of (*66*)). An antisense *Wisper* transcript was used as control. Then, proteins were identified by shotgun mass spectrometry. Four proteins were detected as specifically associated with *Wisper*, namely TIAR, PTB3, DIS3L2 and CELF2 (Fig. 5A). All four RNA binding proteins have been implicated in RNA processing. TIAR, PTBP3 and CELF2 are splicing factors, and DIS3L2 has been involved in target mRNA-mediated microRNA degradation as well as mRNA decay (*39-43*). These proteins demonstrate relevant functions as regulators of differentiation, proliferation and apoptosis during development and in adulthood. Nevertheless, protein inference based on peptide analysis unambiguously identified TIAR (TIA1-related protein also referred to as TIA1 cytotoxic granule-associated RNA binding protein-like 1 or TIAL1) as a prime candidate with 26% amino acid coverage (102/392). More importantly, TIAR has been related to tissue fibrosis via its capacity to regulate expression of lysyl hydroxylase 2 (also known as procollagen lysine, 2-oxoglutarate 5-dioxygenase or *Plod2*) (*44*), and thereby the extent of collagen cross-linking. We confirmed the strong association of TIAR with *Wisper* using Western blotting to detect TIAR following *Wisper* pulldown (Fig. 5B). Then, we performed a RNA immunoprecipitation assay to validate *Wisper*-TIAR interaction (Fig. 5C). Real-time PCR following TIAR immunoprecipitation detected *Wisper* and *Plod2* as TIAR bound-transcripts but not *Wisp2.* Importantly, *Wisper* knockdown reduced the amounts of TIAR associated-*Wisper* as expected, and in addition also affected the amounts of TIAR bound-*Plod2* (Fig. 5C). Downregulation of *Wisper* expression in cardiac fibroblasts resulted therefore in decreased *Plod2* expression whereas *Wisp2* depletion did not change *Plod2* concentrations (Fig. 5D). TIAR has been demonstrated to shuttle between the cytoplasm and the nucleus (*45*). Since Wisper was also found in both the cytoplasm and the nucleus, we investigated whether TIAR nuclear translocation could depend on *Wisper* action. Primary cardiac fibroblasts were therefore transfected with *Wisper*-targeting GapmeRs and TIAR subcellular localization was determined by immunostaining (Fig. 5E). Wisper knockdown resulted in a dose-dependent decrease in the number of fibroblasts with nuclear TIAR staining. Confocal microscopy confirmed the absence of TIAR in the nucleus and retention of the protein in the cytoplasm of treated cells.

### Preventive Wisper depletion in vivo inhibits cardiac fibrosis

To test if the anti-fibrotic effects of *Wisper* in cultured CFs may hold therapeutic potential in cardiac fibrosis, we performed loss of function experiments in mice. We first completed a dose escalation study in adult untouched mice by administering 5, 10 and 15 mg/kg of *Wisper* GapmeRs (Fig. S5A of (*66*)). Control mice received a scrambled GapmeR at a dose of 10 mg/kg. All mice injected with 15 mg kg died within the first week following GapmeR injection, whereas the 5 and 10 mg/kg doses had no impact on survival (Fig. S5B of (*66*)). Echocardiography performed at 4, 14, and 28 days post injection showed that mice receiving 10 mg/kg of *Wisper* GapmeRs demonstrated signs of cardiac remodeling, in particular a thickening of the interventricular septum. Animals injected with 5 mg/kg were not different from controls (Fig. S5C & Table S2 of (*66*)). To determine the impact of acute and massive *Wisper* depletion on cardiac dimensions and function, echocardiography was performed in untouched mice receiving 15 mg/kg *Wisper* GapmeR four days after injection. At this time point, the myocardium exhibited structural alterations, including an increased thickness of the ventricular septum and of the posterior wall, with a concomitant reduction of the left ventricular cavity. This peculiar situation was characterized by a paradoxical increase in ejection fraction, associated to a largely decreased stroke volume, creating a very detrimental situation. (Fig. S5D of (66)). *Wisper* was significantly depleted in isolated adult CFs (*P* < 0.007), and a robust downregulation of key PCGs, including *Col1a1*, *Col3a1*, *Vim* and *Postn* was also observed (Fig. S5E-F of (*66*)). Finally, a significant upregulation of cardiac stress markers, including *Ctgf* and *Myh7* (*P =* 0.012 and 0.011 respectively), was measured. Interestingly, despite a dramatic decrease of collagen expression in the heart in animals receiving this toxic dose of *Wisper* GapmeRs, no effects on collagen expression were observed in the kidneys or the liver (Fig. S5G of (*66*)). Signs of liver damage were however evident since plasma concentrations of both aspartate aminotransferase (AST) and alanine aminotransferase (ALT) were elevated (Fig. S5H of (*66*)). Importantly, the amounts of these enzymes in the blood of mice receiving 5 mg/kg were not changed, indicating no toxic effects of Wisper GapmeR at this concentration. Based on these data the 5 mg/kg dose was therefore selected for subsequent experiments in vivo.

We used GapmeRs to perturb the induction of *Wisper* in a preventive strategy by delivering scrambled and *Wisper*-targeting GapmeRs 3 days prior to the induction of MI, and assessed cardiac function 7 and 14 days after MI (Fig. S6A of (*66*)). RNA was isolated and histological sections were generated 14 days post-infarction. Consistent with the observed effects in vitro, delivery of *Wisper* GapmeR resulted in the blunted expression of *Wisper* in the heart (Fig. S6B of (66)) and of key ECM-associated PCGs (Fig. S6C of (*66*)). *Wisper* was not affected by GapmeR treatment in sham-operated animals, suggesting that only stress-stimulated *Wisper* expression was sensitive to knockdown using a dose of 5 mg/kg. Importantly, gene expression was measured more than 2 weeks after GapmeR administration. Compared to scrambled GapmeR-treated animals, mice treated with *Wisper* GapmeR exhibited improved structural and functional parameters at 7 and 14 days post-MI as assessed by echocardiography (Fig. S6D and E as well as Table S3 of (*66*)). *Wisper-depleted* mice demonstrated decreased remodeling as indicated from decreased heart weight to tibial length ratio (Fig. S6F of (*66*)). Infarct size was reduced and a significant decrease in myocardial fibrosis was observed (Fig. S6G of (*66*); *P =* 0.006). Considering the impact of *Wisper* depletion on CF proliferation in vitro, we also assessed non-myocyte cell proliferation in vivo via BrdU incorporation. The reduced numbers of BrdU-positive non-myocyte cells in *Wisper* GapmeR-treated animals were indicative of decreased proliferation in this subpopulation (Fig. S6H of (*66*)). However, despite these largely beneficial effects of *Wisper* depletion on maladaptive remodeling, decreased CF survival prior to injury may also be expected to affect the acute wound healing process. Indeed, we observed that *Wisper* GapmeR-treated mice exhibited a higher rate of mortality during the acute phase after MI, primarily resulting from left ventricular wall rupture, suggesting that *Wisper* silencing might impair the acute would healing process that relies on CF activity (Fig. 11). This prompted us to test the therapeutic potential of *Wisper* depletion in a therapeutic protocol more relevant for a clinical setting.

### Therapeutic depletion of Wisper in vivo inhibits cardiac fibrosis and improves function

To evaluate the potential utility of *Wisper* targeting as an anti-fibrotic therapy, we utilized a therapeutic protocol in which *Wisper* was depleted after MI. MI was induced and *Wisper* GapmeRs were injected 2 and 9 days post-injury to avoid impacting acute wound healing but to subsequently modulate the evolution of the pathological proliferative phase of the remodeling process (Fig. 6A). Cardiac dimensions and function were assessed by echocardiography at 7 and 28 days post-MI. RNA was isolated upon sacrifice at 28 days, a temporal point coinciding with the evolution of the mature scar and the development of pathological remodeling. Both *Wisper* and its proximal PCG *Wisp2* demonstrated blunted cardiac expression in *Wisper* GapmeR-treated mice (Fig. 6A). This profile was associated with significant impact on the expression of key ECM and pro-fibrotic PCGs including *Tgfb2* (*P* = 0.003), *Col1a1* (*P =* 0.007), *Col3a1* (*P =* 0.010), *Fn1* (*P* = 0.010) and *a-SMA* (*P* = 0.021) (Fig. 6B; Fig. S7A of (*66*)). Additionally, expression of cardiac stress markers was also blunted upon *Wisper* silencing suggestive of a beneficial impact on cardiac hypertrophy (Fig. S7A of (*66*)). At both 7 and 28 days post-MI, *Wisper*-depleted mice exhibited significantly improved cardiac function (Fig. 6E; %FS: *P* = 0.008 and 0.004 at 7 and 28 days respectively), and decreased remodeling (Fig. 6C-E and Table S4 of (*66*)). This response was associated with a reduction in infarct size and a significant perturbation of cardiac fibrosis (Fig. 6F; *P* = 0.002), resulting in preserved tissue architecture and reduced thinning of the myocardial wall after *Wisper* knockdown. In support of this, post infarction fibrosis was found highly correlated with *Wisper* expression in treated mice, even better correlated than with the expression of canonical ECM associated PCGs such as *Col1a1* and *Col3a1* ((*66*)). Importantly, mortality rate in treated animals was not augmented during the acute phase, indicating that the wound healing process was not negatively impacted (Fig. 6G). *Wisper-*depleted mice had a slightly increased survival rate post-injury when compared to controls, suggestive of an overall beneficial effect on mortality rates. Collectively these data support an important role for *Wisper* in pathological cardiac fibrosis and demonstrate that therapeutic targeting of *Wisper* after MI elicits clinically desirable effects.

### WISPER expression correlates with the extent of fibrosis in the diseased human heart

A putative ortholog of *Wisper* was identified in the human genome, mapping to a left ventricle-specific SE (Fig. 8 and (*66*)). Primers were designed to amplify this transcript, which could be consistently detected in RNA isolated from human cardiac biopsies, formally demonstrating that *WISPER* was evolutionary conserved. We therefore proceeded to quantify *WISPER* expression in RNA isolated from the interventricular septum of patients suffering from aortic stenosis (AOS). AOS is associated with extensive myocardial fibrosis that directly contributes to left ventricle dysfunction. In cardiac samples from all AOS patients, the collagen volume fraction (CVF) was higher than in samples from healthy volunteers (*46*). After analyzing the distribution of CVF in the AOS cohort, two groups were identified: a group with non-severe fibrosis (n=11) with a CVF lower than 12%, and a severe fibrosis group (n=15) with a CVF greater than 12%. Interestingly, *WISPER* expression, and not the expression of its proximal PCG *WISP2,* was significantly increased in the severe fibrosis group (Fig. 7A; *P* = 0.012 and 0.120 respectively). Moreover, in a correlation analysis, *WISPER* expression and not *WISP2* was found to be associated with the degree of CVF in all AOS patients (Fig. 7B). To evaluate the functional importance of *WISPER* in human fibroblasts, we used human dermal fibroblasts and human CFs. Both cell types can be induced to differentiate into myofibroblasts via serum starvation. This process is associated with the upregulation of *COL1A1, COL3A1* and *αSMA* in both fibroblast populations (*66*). Interestingly, *WISPER* was significantly induced by serum starvation in CFs but not in dermal fibroblasts (Fig. 7C; *P* < 0.001). *WISP2* was also upregulated in differentiating CFs. Supporting observations in the mouse, *WISPER* expression was correlated with *COL3A1*, *FN1* and *αSMA* expression in differentiating human CFs (Fig. 7D). The functional importance of *WISPER* was tested in knockdown experiments. *WISPER* depletion using GapmeRs resulted in decreased expression of *COL1A1*, *COL3A1*, *FN1* and *αSMA* in human CFs (Fig. 7E). Silencing of *WISPER* did not impact *WISP2* expression, suggesting again that *WISPER* controls fibrotic gene expression independently of *WISP2.* Finally, *PLOD2* expression was decreased in human CFs following GapmeR-mediated *WISPER* depletion (Fig. 7F). These findings demonstrate that *WISPER* is an lncRNA conserved in human, and highlight the translational relevance of *WISPER* as a therapeutic target in cardiac fibrosis.

**Table 1**

| **Sequences** | | **SEQ ID No.** |
|---|---|---|
| Human Proximal Wisper-Enhancer | hg38_dna range=chr20:44691595-44696953 5'pad=0 | 1 |
| | 3'pad=0 strand=+ | |
| Human Wisper Proximal Promoter | hg19_dna range=chr20:43324240-43329987 5'pad=0 | 2 |
| | 3'pad=0 strand=+ | |
| Human WISPER Super-enhancer | hg19_dna range=chr20:43318059-43342644 5'pad=0 | 3 |
| | 3'pad=0 strand=+ | |
| Human WISPER Downstream cis-sequences | hg19_dna range=chr20:43283796-43318224 5'pad=0 | 4 |
| | 3'pad=0 strand=+ | |
| SMAD7- cis-regulatory | lncRNA XLOC_015277 hg19_dna | 5 |
| | range=chr18:46477041-46490607 5'pad=0 3'pad=0 | |
| | strand=+ | |
| CYR61 cis-regulatory | hg19_dna range=chr1:86071896-86085046 5'pad=0 | 6 |
| | 3'pad=0 strand=+ | |
| COL15A1 cis-regulatory | XLOC_022236 | 7 |
| | hg19_dna range=chr9:101700203-101712895 5'pad=0 | |
| | 3'pad=0 strand=+ | |
| SPARC cis-regulatory | XLOC004910 | 8 |
| | _ hg19_dna range=chr5:151000095-151012897 5'pad=0 | |
| | 3'pad=0 strand=+ | |

**Table 2**

| | **Fibrosis Group** | |
|---|---|---|
| | **Non Severe** | **Severe** |
| **n** | 11 | 15 |
| **CVF(Avg.)** | 7.40 | 26.38 |
| **Age (Avg.)** | 71 | 71.47 |
| **Gender** | 10 Females; 1 Males | 8 Females; 7 Males |
| **Hypertension** | 8 Yes; 3 No | 11 Yes; 4 No |
| **Atrial fibrillation** | 2 Yes; 9 No | 8 Yes; 7 No |
| **BMI (Avg.)** | 28.95 | 28.61 |
| **SBP (Avg.) (mm Hg)** | 121.82 | 118.53 |
| **DBP (Avg.) (mm Hg)** | 71.09 | 67.67 |
| **HR (Avg.) (beats/min)** | 72.09 | 77.47 |
| **LVMI (Avg.)** | 125.57 | 155.15 |
| **LVH** | 5 Yes; 6 No | 9 Yes; 6 No |
| **RWT (Avg.)** | 0.62 | 0.62 |
| **AVAi(Avg.)** | 0.32 | 0.39 |
| **LVEDD (Avg.)** | 4.14 | 4.66 |
| **LVESD(Avg.)** | 2.29 | 3.11 |
| **LVEF(Avg.)** | 75.44 | 60.72 |
| **DT(Avg.)** | 290.91 | 269.13 |
| **IVRT(Avg.)** | 84.00 | 119.13 |

| | | |
|---|---|---|
| BMI, body mass index; SBP, systolic blood pressure DBP, diastolic blood pressure HR, heart rate; LVMI, left ventricular mass index (g/m2) LVH, LV hypertrophy; RWT, relative wall thickness AVAi, aortic valve area index LVEDD, LV end-diastolic diameter LVESD, LV end-systolic diameter; LVEF, left ventricular ejection fraction DT, deceleration time; IVRT, isovolumic relaxation time. | | |

**Table 3**

| **GapmeRs Sequences** | | | | **SEQ ID No.** |
|---|---|---|---|---|
| **Species** | **GeneName** | **Sequence(5'---3')** | **Supplier** | |
| both | Negative CTRLA | AACACGTCTATACGC | Exiqon | 9 |
| mouse | Mm_Wisper | AGGTGTGCGATAGAG | Exiqon | 10 |
| mouse | Mm_Wisp2 | CGCAAGTGCCAAGAGT | Exiqon | 11 |
| human | Hu_WISPER | CAAGAAGCTGGAGTTG | Exiqon | 12 |
| human | Hu_WISPER_2 | AGGTGTGCGA TAGAG | Exiqon | 14 |

### References

1. S. D. Prabhu, N. G. Frangogiannis, The Biological Basis for Cardiac Repair After Myocardial Infarction: From Inflammation to Fibrosis. Circ Res 119, 91-112 (2016).
2. N. G. Frangogiannis, Pathophysiology of Myocardial Infarction. Compr Physiol 5, 1841-1875 (2015).
3. M. Writing Group, D. Mozaffarian, E. J. Benjamin, A. S. Go, D. K. Arnett, M. J. Blaha, M. Cushman, S. R. Das, S. de Ferranti, J. P. Despres, H. J. Fullerton, V. J. Howard, M. D. Huffman, C. R. Isasi, M. C. Jimenez, S. E. Judd, B. M. Kissela, J. H. Lichtman, L. D. Lisabeth, S. Liu, R. H. Mackey, D. J. Magid, D. K. McGuire, E. R. Mohler, 3rd, C. S. Moy, P. Muntner, M. E. Mussolino, K. Nasir, R. W. Neumar, G. Nichol, L. Palaniappan, D. K. Pandey, M. J. Reeves, C. J. Rodriguez, W. Rosamond, P. D. Sorlie, J. Stein, A. Towfighi, T. N. Turan, S. S. Virani, D. Woo, R. W. Yeh, M. B. Turner, C. American Heart Association Statistics, S. Stroke Statistics, Heart Disease and Stroke Statistics-2016 Update: A Report From the American Heart Association. Circulation 133, e38-60 (2016).
4. F. G. Spinale, M. R. Zile, Integrating the myocardial matrix into heart failure recognition and management. Circ Res 113, 725-738 (2013).
5. E. B. Schelbert, G. C. Fonarow, R. O. Bonow, J. Butler, M. Gheorghiade, Therapeutic targets in heart failure: refocusing on the myocardial interstitium. J Am Coll Cardiol 63, 2188-2198 (2014).
6. K. T. Weber, Y. Sun, S. K. Bhattacharya, R. A. Ahokas, I. C. Gerling, Myofibroblast-mediated mechanisms of pathological remodelling of the heart. Nat Rev Cardiol 10, 15-26 (2013).
7. J. K. Lighthouse, E. M. Small, Transcriptional control of cardiac fibroblast plasticity. J Mol Cell Cardiol 91, 52-60 (2016).
8. K. B. Schuetze, T. A. McKinsey, C. S. Long, Targeting cardiac fibroblasts to treat fibrosis of the heart: focus on HDACs. J Mol Cell Cardiol 70, 100-107 (2014).
10. S. Ounzain, T. Pedrazzini, Super-enhancer lncs to cardiovascular development and disease. Biochim Biophys Acta 1863, 1953-1960 (2016).
11. J. A. Wamstad, X. Wang, O. O. Demuren, L. A. Boyer, Distal enhancers: new insights into heart development and disease. Trends Cell Biol 24, 294-302 (2014).
12. D. Hnisz, B. J. Abraham, T. I. Lee, A. Lau, V. Saint-Andre, A. A. Sigova, H. A. Hoke, R. A. Young, Super-enhancers in the control of cell identity and disease. Cell 155, 934-947 (2013).
13. W. A. Whyte, D. A. Orlando, D. Hnisz, B. J. Abraham, C. Y. Lin, M. H. Kagey, P. B. Rahl, T. I. Lee, R. A. Young, Master transcription factors and mediator establish super-enhancers at key cell identity genes. Cell 153, 307-319 (2013).
15. K. V. Morris, J. S. Mattick, The rise of regulatory RNA. Nat Rev Genet 15, 423-437 (2014).
18. R. A. Boon, S. Dimmeler, MicroRNAs in myocardial infarction. Nat Rev Cardiol 12, 135-142 (2015).
22. K. Wang, F. Liu, L. Y. Zhou, B. Long, S. M. Yuan, Y. Wang, C. Y. Liu, T. Sun, X. J. Zhang, P. F. Li, The long noncoding RNA CHRF regulates cardiac hypertrophy by targeting miR-489. Circ Res 114, 1377-1388 (2014).
23. M. T. Piccoli, C. Bar, T. Thum, Non-coding RNAs as modulators of the cardiac fibroblast phenotype. J Mol Cell Cardiol 92, 75-81 (2016).
24. T. R. Mercer, J. S. Mattick, Structure and function of long noncoding RNAs in epigenetic regulation. Nat Struct Mol Biol 20, 300-307 (2013).
25. W. Li, D. Notani, M. G. Rosenfeld, Enhancers as non-coding RNA transcription units: recent insights and future perspectives. Nat Rev Genet 17, 207-223 (2016).
26. A. A. Sigova, A. C. Mullen, B. Molinie, S. Gupta, D. A. Orlando, M. G. Guenther, A. E. Almada, C. Lin, P. A. Sharp, C. C. Giallourakis, R. A. Young, Divergent transcription of long noncoding RNA/mRNA gene pairs in embryonic stem cells. Proc Natl Acad Sci USA 110, 2876-2881 (2013).
27. A. A. Sigova, B. J. Abraham, X. Ji, B. Molinie, N. M. Hannett, Y. E. Guo, M. Jangi, C. C. Giallourakis, P. A. Sharp, R. A. Young, Transcription factor trapping by RNA in gene regulatory elements. Science 350, 978-981 (2015).
28. M. Mele, J. L. Rinn, "Cat's Cradling" the 3D Genome by the Act of LncRNA Transcription. Mol Cell 62, 657-664 (2016).
29. S. Ounzain, R. Micheletti, T. Beckmann, B. Schroen, M. Alexanian, I. Pezzuto, S. Crippa, M. Nemir, A. Sarre, R. Johnson, J. Dauvillier, F. Burdet, M. Ibberson, R. Guigo, I. Xenarios, S. Heymans, T. Pedrazzini, Genome-wide profiling of the cardiac transcriptome after myocardial infarction identifies novel heart-specific long non-coding RNAs. Eur Heart J 36, 353-368 (2015).
31. D. Jeong, M. A. Lee, Y. Li, D. K. Yang, C. Kho, J. G. Oh, G. Hong, A. Lee, M. H. Song, T. J. LaRocca, J. Chen, L. Liang, S. Mitsuyama, V. D'Escamard, J. C. Kovacic, T. H. Kwak, R. J. Hajjar, W. J. Park, Matricellular Protein CCN5 Reverses Established Cardiac Fibrosis. J Am Coll Cardiol 67, 1556-1568 (2016).
32. S. Pott, J. D. Lieb, What are super-enhancers? Nat Genet 47, 8-12 (2015).
33. T. Okubo, B. L. Hogan, Hyperactive Wnt signaling changes the developmental potential of embryonic lung endoderm. J Biol 3, 11 (2004).
34. N. G. Frangogiannis, The inflammatory response in myocardial injury, repair, and remodelling. Nat Rev Cardiol 11, 255-265 (2014).
35. P. Wiesel, L. Mazzolai, J. Nussberger, T. Pedrazzini, Two-kidney, one clip and one-kidney, one clip hypertension in mice. Hypertension 29, 1025-1030 (1997).
36. S. Konermann, M. D. Brigham, A. E. Trevino, J. Joung, O. O. Abudayyeh, C. Barcena, P. D. Hsu, N. Habib, J. S. Gootenberg, H. Nishimasu, O. Nureki, F. Zhang, Genome-scale transcriptional activation by an engineered CRISPR-Cas9 complex. Nature 517, 583-588 (2015).
37. F. Yang, F. Yi, X. Han, Q. Du, Z. Liang, MALAT-1 interacts with hnRNP C in cell cycle regulation. FEBS Lett 587, 3175-3181 (2013).
38. E. Hacisuleyman, L. A. Goff, C. Trapnell, A. Williams, J. Henao-Mejia, L. Sun, P. McClanahan, D. G. Hendrickson, M. Sauvageau, D. R. Kelley, M. Morse, J. Engreitz, E. S. Lander, M. Guttman, H. F. Lodish, R. Flavell, A. Raj, J. L. Rinn, Topological organization of multi chromosomal regions by the long intergenic noncoding RNA Firre. Nat Struct Mol Biol 21, 198-206 (2014).
39. C. Le Guiner, F. Lejeune, D. Galiana, L. Kister, R. Breathnach, J. Stevenin, F. Del Gatto-Konczak, TIA-1 and TIAR activate splicing of alternative exons with weak 5' splice sites followed by a U-rich stretch on their own pre-mRNAs. J Biol Chem 276, 40638-40646 (2001).
40. S. Waris, M. C. Wilce, J. A. Wilce, RNA recognition and stress granule formation by TIA proteins. Int J Mol Sci 15, 23377-23388 (2014).
41. L. Y. Tan, P. Whitfield, M. Llorian, E. Monzon-Casanova, M. D. Diaz-Munoz, M. Turner, C. W. Smith, Generation of functionally distinct isoforms of PTBP3 by alternative splicing and translation initiation. Nucleic Acids Res 43, 5586-5600 (2015).
42. Y. Blech-Hermoni, S. J. Stillwagon, A. N. Ladd, Diversity and conservation of CELF1 and CELF2 RNA and protein expression patterns during embryonic development. Dev Dyn 242, 767-777 (2013).
43. G. Haas, S. Cetin, M. Messmer, B. Chane-Woon-Ming, O. Terenzi, J. Chicher, L. Kuhn, P. Hammann, S. Pfeffer, Identification of factors involved in target RNA-directed microRNA degradation. Nucleic Acids Res 44, 2873-2887 (2016).
44. H. N. Yeowell, L. C. Walker, D. M. Mauger, P. Seth, M. A. Garcia-Blanco, TIA nuclear proteins regulate the alternate splicing of lysyl hydroxylase 2. J Invest Dermatol 129, 1402-1411 (2009).
45. T. Zhang, N. Delestienne, G. Huez, V. Kruys, C. Gueydan, Identification of the sequence determinants mediating the nucleo-cytoplasmic shuttling of TIAR and TIA-1 RNA-binding proteins. J Cell Sci 118, 5453-5463 (2005).
46. J. Beaumont, B. Lopez, N. Hermida, B. Schroen, G. San Jose, S. Heymans, F. Valencia, J. J. Gomez-Doblas, E. De Teresa, J. Diez, A. Gonzalez, microRNA-122 down-regulation may play a role in severe myocardial fibrosis in human aortic stenosis through TGF-beta1 up-regulation. Clin Sci (Lond) 126, 497-506 (2014).
47. G. Rizki, L. A. Boyer, Lncing Epigenetic Control of Transcription to Cardiovascular Development and Disease. Circ Res 117, 192-206 (2015).
49. C. Sanchez-Jimenez, J. M. Izquierdo, T-cell intracellular antigens in health and disease. Cell Cycle 14, 2033-2043 (2015).
50. J. Wu, D. P. Reinhardt, C. Batmunkh, W. Lindenmaier, R. K. Far, H. Notbohm, N. Hunzelmann, J. Brinckmann, Functional diversity of lysyl hydroxylase 2 in collagen synthesis of human dermal fibroblasts. Exp Cell Res 312, 3485-3494 (2006).
51. Z. Wang, M. Kayikci, M. Briese, K. Zarnack, N. M. Luscombe, G. Rot, B. Zupan, T. Curk, J. Ule, iCLIP predicts the dual splicing effects of TIA-RNA interactions. PLoS Biol 8, e1000530 (2010).
52. L. Liu, H. Yue, Q. Liu, J. Yuan, J. Li, G. Wei, X. Chen, Y. Lu, M. Guo, J. Luo, R. Chen, LncRNA MT1JP functions as a tumor suppressor by interacting with TIAR to modulate the p53 pathway. Oncotarget 7, 15787-15800 (2016).
53. C. Sanchez-Jimenez, J. M. Izquierdo, T-cell intracellular antigen (TIA)-proteins deficiency in murine embryonic fibroblasts alters cell cycle progression and induces autophagy. PLoS One 8, e75127 (2013).
55. R. Kumarswamy, C. Bauters, I. Volkmann, F. Maury, J. Fetisch, A. Holzmann, G. Lemesle, P. de Groote, F. Pinet, T. Thum, Circulating long noncoding RNA, LIPCAR, predicts survival in patients with heart failure. Circ Res 114, 1569-1575 (2014).
56. B. E. Bernstein, J. A. Stamatoyannopoulos, J. F. Costello, B. Ren, A. Milosavljevic, A. Meissner, M. Kellis, M. A. Marra, A. L. Beaudet, J. R. Ecker, P. J. Farnham, M. Hirst, E. S. Lander, T. S. Mikkelsen, J. A. Thomson, The NIH Roadmap Epigenomics Mapping Consortium. Nat Biotechnol 28, 1045-1048 (2010).
57. L. H. Michael, M. L. Entman, C. J. Hartley, K. A. Youker, J. Zhu, S. R. Hall, H. K. Hawkins, K. Berens, C. M. Ballantyne, Myocardial ischemia and reperfusion: a murine model. Am J Physiol 269, H2147-2154 (1995).
58. B. Lopez, R. Querejeta, A. Gonzalez, M. Larman, J. Diez, Collagen cross-linking but not collagen amount associates with elevated filling pressures in hypertensive patients with stage C heart failure: potential role of lysyl oxidase. Hypertension 60, 677-683 (2012).
59. A. Dobin, C. A. Davis, F. Schlesinger, J. Drenkow, C. Zaleski, S. Jha, P. Batut, M. Chaisson, T. R. Gingeras, STAR: ultrafast universal RNA-seq aligner. Bioinformatics 29, 15-21 (2013).
60. S. Anders, P. T. Pyl, W. Huber, HTSeq--a Python framework to work with high-throughput sequencing data. Bioinformatics 31, 166-169 (2015).
61. L. Wang, S. Wang, W. Li, RSeQC: quality control of RNA-seq experiments. Bioinformatics 28, 2184-2185 (2012).
62. B. Li, C. N. Dewey, RSEM: accurate transcript quantification from RNA-Seq data with or without a reference genome. BMC Bioinformatics 12, 323 (2011).
63. M. D. Robinson, D. J. McCarthy, G. K. Smyth, edgeR: a Bioconductor package for differential expression analysis of digital gene expression data. Bioinformatics 26, 139-140 (2010).
64. M. E. Ritchie, B. Phipson, D. Wu, Y. Hu, C. W. Law, W. Shi, G. K. Smyth, limma powers differential expression analyses for RNA-sequencing and microarray studies. Nucleic Acids Res 43, e47 (2015).
65. Y. Benjamini, Y. Hochberg, Controlling the false discovery rate: a practical and powerful approach to multiple testing. Journal of the Royal Statistical Society Series B 57, 289-300 (1995).
66. Micheletti R, Plaisance I, Abraham BJ, Sarre A, Ting CC, Alexanian M, Maric D, Maison D, Nemir M, Young RA, Schroen B, González A, Ounzain S, Pedrazzini T. The long noncoding RNA Wisper controls cardiac fibrosis and remodeling Sci Transl Med. 2017 Jun 21; 9(395).
67. Dominguez AA, Lim WA, and Qi LS. Beyond editing: repurposing CRISPR-Cas9 for precision genome regulation and interrogation. Nat Rev Mol Cell Biol. 2016 Jan;17(1):5-15.
68. Andriy Didovyk, Bart 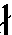 omiej Borek, Lev Tsimring, and Jeff Hasty. Curr Opin Biotechnol. 2016 Aug; 40: 177-184.

## Claims

1. A nucleic acid comprising an antisense oligonucleotide, miRNA, siRNA, piRNA, hnRNA, snRNA, esiRNA, or shRNA , targeting a regulatory sequence of one or more lncRNA associated with cardiac-specific super-enhancers (SEs), wherein the one or more lcRNA associated with cardiac-specific super-enhancers (SEs) is selected from the group consisting of *Wisper, Sparc, Col15A1, Cyr61, Smad7* and a combination of one or more thereof, and wherein the regulatory sequence is a promoter or an enhancer sequence selected from the group comprising the proximal Wisper enhancer (SEQ ID No. 1), the proximal Wisper promoter (SEQ ID No. 2 ), the Wisper super-enhancer (SEQ ID No. 3), the Wisper downstream cis sequence (SEQ ID No. 4), the Smad7 cis-regulatory sequence (SEQ ID No. 5), the Cyr61 cis-regulatory sequence (SEQ ID No. 6 ), the Col15A1 cis-regulatory sequence (SEQ ID No.7 ), the Sparc cis-regulatory sequence (SEQ ID No. 8) and a combination of one or more thereof.

2. The nucleic acid of claim 1, wherein the nucleic acid is the antisense oligonucleotide, and wherein the antisense oligonucleotide is a GapmeR.

3. The nucleic acid of claim 2, wherein the antisense oligonucleotide comprises one or more locked nucleic acids (LNAs).

4. The nucleic acid of claim 2 or claim 3, wherein the antisense oligonucleotide is 16 to 25 nucleotides in length

5. A pharmaceutical composition comprising the nucleic acid of any one of claims 1-4, optionally with one or more pharmaceutically acceptable excipient or carrier.

6. The pharmaceutical composition of claim 5 for use in the treatment and/or prevention of a cardiac diseasein a subject.

7. The pharmaceutical composition of claim 6, wherein the subject suffers from or is at risk of suffering from the cardiac disease.

8. The pharmaceutical composition of claim 6 or claim 7, wherein the cardiac disease is myocardial infarction (MI), heart failure, cardiomyopathies, cardiac fibrosis, or heart remodeling.
